# EUROPEAN PATENT SPECIFICATION

(11) **EP 3 423 106 B1**
(45) Date of publication and mention of the grant of the patent: **27.07.2022**
(21) Application number: 17711856.9
(22) Date of filing: 28.02.2017
(51) Int. Cl.: A61K 47/59, C12N 15/87, A61K 48/00, C08G 63/91, C08G 63/00

(54) **BIODEGRADABLE ACTIVATED POLYMERS FOR THERAPEUTIC DELIVERY**
BIOLOGISCH ABBAUBARE AKTIVIERTE POLYMERE ZUR THERAPEUTISCHEN VERABREICHUNG
POLYMÈRES ACTIVÉS BIODÉGRADABLES POUR ADMINISTRATION THÉRAPEUTIQUE

(30) Priority: 01.03.2016 US 201662301916 P; 15.03.2016 US 201662308319 P
(43) Date of publication of application: 09.01.2019
(73) Proprietor: Alexion Pharmaceuticals, Inc., New Haven, CT 06510 (US); Yale University, New Haven, CT 06510 (US)
(72) Inventor: CHENG, Christopher, J., New Haven, CT 06510 (US); SALTZMAN, W., Mark, New Haven, CT 06510 (US); ZHANG, Junwei, New Haven, CT 06510 (US)
(74) Representative: Murgitroyd & Company
(86) International application number: PCT/US2017/019962
(87) International publication number: WO 2017/151623

(56) References cited:
- US-A1- 2015 073 041

## Description

### BACKGROUND

Gene therapy strategies are promising for the treatment of numerous chronic diseases. DNA-based treatments, however, often carry safety risks and can lack an ability to control protein expression. mRNA-based therapies often address those concerns but require an efficient delivery method that is safe enough for chronic use.

Non-viral vehicles for gene delivery are known for their limited immunogenicity, ability to accommodate and deliver macromolecules, and potential for surface modification. Major categories of non-viral delivery vehicles include cationic lipids and cationic polymers. Both cationic lipid and cationic polymer systems deliver genes by forming condensed complexes with negatively charged DNA through electrostatic interactions. These complexes protect the DNA from degradation and facilitate its cellular uptake and intracellular traffic into the nucleus.

Polyplexes formed between cationic polymers and DNA are generally more stable than lipoplexes formed between cationic lipids and DNA, but both are often unstable in physiological fluids due to serum components and salts, which tend to cause the complexes to break apart or aggregate. Transfection by both lipids and polymers, furthermore, usually requires materials with excess charge, resulting in polyplexes or lipoplexes with net positive charges on the surface. When injected into the circulatory system, the positive surface charge initiates rapid formation of complex aggregates with negatively charged serum molecules or membranes of cellular components, which are then cleared by the reticuloendothelial system (RES).

Many of the cationic polyplexes or lipoplexes developed so far have been associated with substantial toxicity, which limits their clinical applicability. Thus, there exists a need for improved non-viral delivery methods for DNA and other nucleic acids *in vivo.*

US 2015/073041 discloses biodegradable polymers, PACE and PACEO, formed by combining PDL and DES, specifically a two-stage process for the preparation of quaterpolymers from a PDL, DES, MEDE and three ortho esters. Although, the average molecular weight (Mw) of the exemplified PACEO polymers is stated to range from 1kDA to 21kDa, the molecular weight of the control polymer PACE and any impact thereof is not disclosed in US 2015/073041. Additionally, the use of biodegradable poly(amine-co-ester) (PACE) terpolymers for efficient DNA delivery can be used for macromolecular delivery. However, injection of polyplexes of 20% PDL-DES-MDEA terpolymer with luciferase plasmid through the tail vein of mice bearing A549-derived tumor xenografts resulted in limited expression of luciferase in the tumors. Further studies suggested the polyplexes were instable in serum, thus limiting their effectiveness. Additionally, the size of these polymers make them unsuitable for gene delivery to the central nervous system.

Thus, there remains a need for improved biodegradable PACE terpolymers that can efficiently and safely deliver macromolecules, including DNA, mRNA and other nucleic acids *in vivo.* There also exists a need for non-viral vectors that can cross the blood-brain barrier (BBB).

### SUMMARY

It will be appreciated that the scope is in accordance with the claims. Accordingly, there is provided an activated polymer as defined in claim 1; a nanoparticle or microparticle comprising the activated polymer as defined in claim 5; a method for activating a polymer as defined in claim 9; a method of making an activated poly(amine co ester) polymer as defined in claim 10; a macromolecule formulated in a particle comprising an activated polymer as defined in claim 11; and an *in-vitro* method of transfecting cells as defined in claim 16. Further features are provided in accordance with the dependant claims. The specification may include description of arrangement outside the scope of the claims provided as background and to assist the understanding of the invention. Accordingly, described herein are methods for the chemical modification of polymers with at least one backbone ester, said modification accomplished by a process comprising exposing the polymer to conditions such that one or more backbone esters are hydrolyzed, thereby exposing one or more activated end group(s).

In particular, methods for the chemical modification of PACE terpolymers that result in novel terpolymers, termed "activated PACE" (aPACE) terpolymers, are disclosed. aPACE terpolymers can be used to efficiently and safely deliver biomolecules, such as DNA, RNA and proteins, both *in vitro* and *in vivo.*

PACE polymers including diesters with various chain length (*e*.*g*., succinate to dodecanedioate) can be copolymerized with diethanolamine with either an alkyl (methyl, ethyl, n-butyl, t-butyl) or an aryl (phenyl) substituent on the nitrogen. One PACE terpolymer, poly (N-methyldiethyleneamine sebacate) (PMSC), transfected a variety of cells *in vitro,* including HEK293, U87-MG, and 9L, with comparable efficiency to leading commercial products (*e*.*g*., LIPOFECTAMINE^{®} 2000 and PEI-14). However, this PACE terpolymer, however, is not effective for systemic delivery of nucleic acids *in vivo* (Wang, Y. et al., Biomacromolecules, 8:1028-37, 2007; Wang, Y. et al., Biomaterials, 28:5358-68, 2007). The activated polymer of the invention as defined in claim 1 is poly(amine co ester) synthesized by a method comprising combining 15 pentadecanolide (PDL), diethanolamine, and a diester/diacid selected from either diethyl sebacate (DES) or sebacic acid (SBA).

Methods for synthesis of PACE terpolymers from lactone, diethyl sebacate (DES) and *N*-methyldiethanolamine (MDEA) using *Candida antarctica* lipase B (CALB) as a catalyst enable the production of PACE terpolymers with diverse chain structures and tunable hydrophobicity. The resulting lactone-DES-MDEA terpolymers range in molecular weight from 18 kDa to 39 kDa and had low nitrogen content (1.9-4.7 wt%). Evaluation of these terpolymers indicated that the PDL-DES-MDEA terpolymer containing 20% lactone displayed the best transfection capability and low toxicity (PDL = 15-pentadecanolide; Zhou, J. et al., Nat. Mater., 11:82-90, 2014). The method of making an activated poly(amine co ester) polymer is defined in claim 10 in which the polymer is poly(amine co ester) synthesized by a method comprising combining 15 pentadecanolide (PDL), diethanolamine, and a diester/diacid selected from either diethyl sebacate (DES) or sebacic acid (SBA),

Also provided are methods for administering a macromolecule, *in vivo* or *in vitro,* comprising, for example, administering the macromolecule in combination with an aPACE polymer described herein (the *in vivo* method as such is not part of the invention as claimed).

### BRIEF DESCRIPTION OF THE DRAWINGS

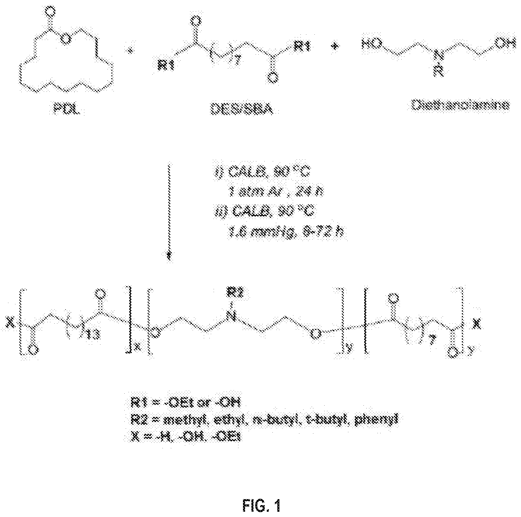

FIG. 1 illustrates a two-stage process for the preparation of terpolymers from 15-pentadecanolide (PDL), diethyl sebacate (DES) / sebacic acid (SBA), and diethanolamine. Values for x and y are as for Formula I.

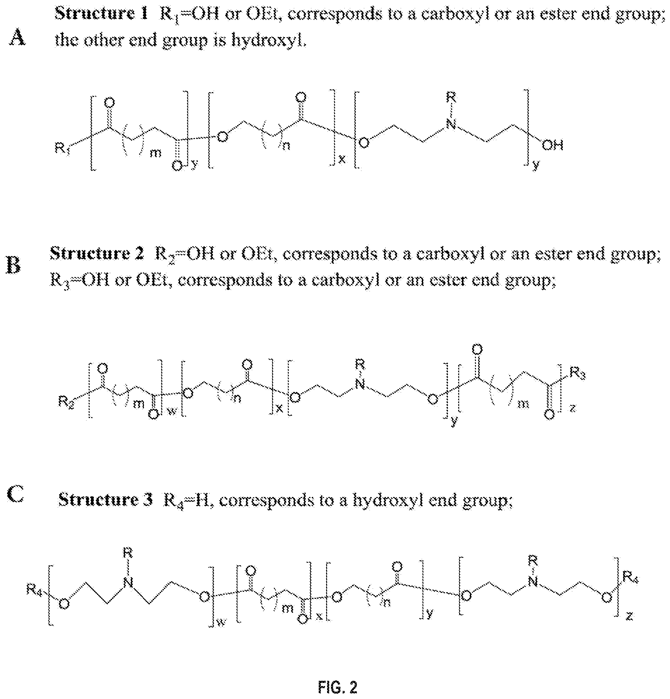

FIG. 2 provides exemplary structures of activated PACE (aPACE) polymers produced by temperature-controlled hydrolysis of PACE polymers. R is as defined for Formula I. Values for m, n, x, y, and z are as for Formula I, and w is an integer from 1-1000. FIG. 2A shows Structure 1; FIG. 2B shows Structure 2; FIG. 2C shows Structure 3.

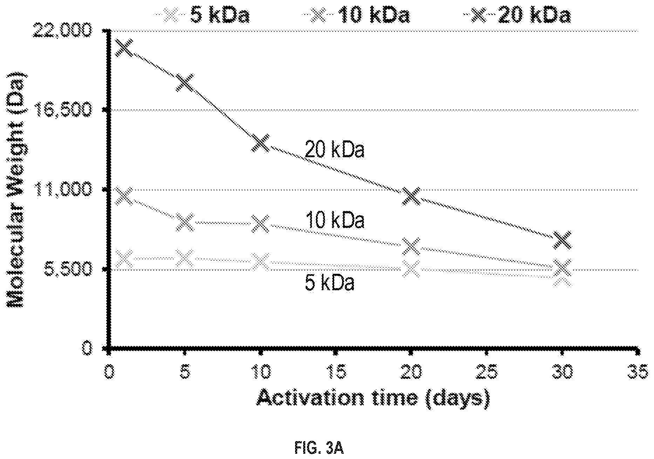

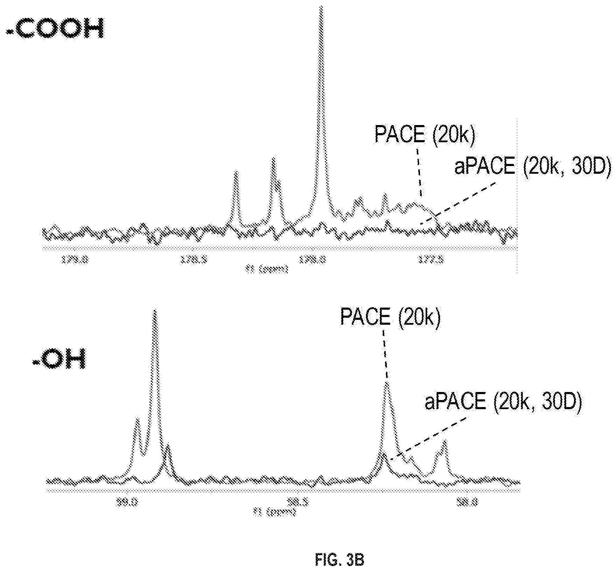

FIG. 3A shows the effect of activation time on molecular weight of the activated PACE terpolymer. Three PACE polymers of differing molecular weights (5 kDa, 10 kDa and 20 kDa) were activated for up to 30 days. Activation of 5 kDa PACE causes slight reduction in molecular weight. Activation of 10 kDa and 20 kDa PACE, however, dramatically reduces molecular weight over the course of 30 days. FIG. 3B shows chemical analysis of 20 kDa PACE terpolymers with either a -COOH end group or -OH end group in comparison with the same PACE terpolymers after activation for 30 days, as measured by inverse gated ¹³C-NMR with Chromium(III) acetylacetonate.

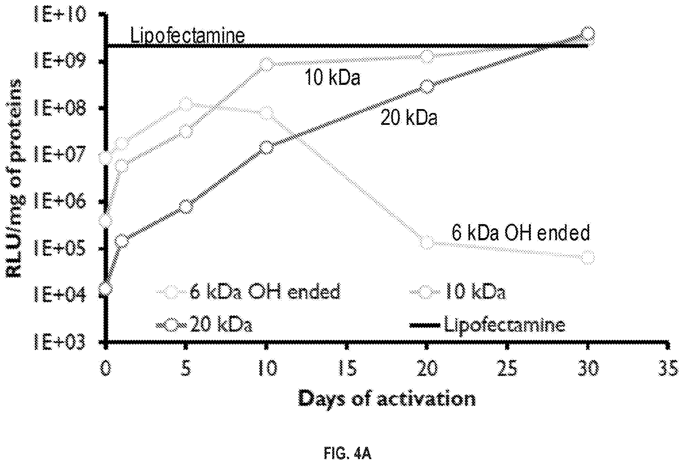

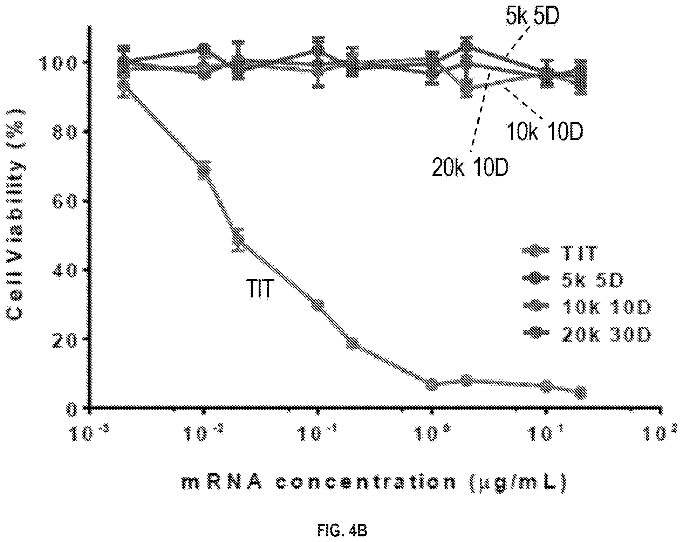

FIG. 4A is a line graph showing that activation of PACE terpolymers increases mRNA transfection efficiency. Three PACE polymers of differing molecular weights (5 kDa; 10 kDa and 20 kDa) were activated for up to 30 days. HEK293 cells were transfected with luciferase mRNA using one of the following: PACE polymers; activated PACE polymers or TRANS-IT^{®} reagent (Mirus Bio LLC). Luciferase production was measured 24 hours after transfection. Without activation, PACE polymers did not transfect HEK293 cells as efficiently as LIPOFECTAMINE, with the larger molecular weight PACE polymers being least effective. Activation of 5 kDa PACE for up to 10 days resulted in increased transfection efficiency. Activation of 10 kDa PACE and 20 kDa PACE for up to 30 days resulted in increased transfection efficiency such that both are indistinguishable from LIPOFECTAMINE. FIG. 4B is a line graph demonstrating that aPACE is non-toxic *in vitro.* Three aPACE polymers (5 kDa PACE activated for 5 days; 10 kDa PACE activated for 10 days; and 20 kDa PACE activated for 30 days) and TRANS-IT^{®} reagent (Mirus Bio LLC) were used to transfect mRNA into HEK293 cells *in vitro.* Toxicity was measured with an MTT assay 24 hours after transfection. All aPACE terpolymers tested were non-toxic when used to transfect up to 20 µg mRNA/mL.

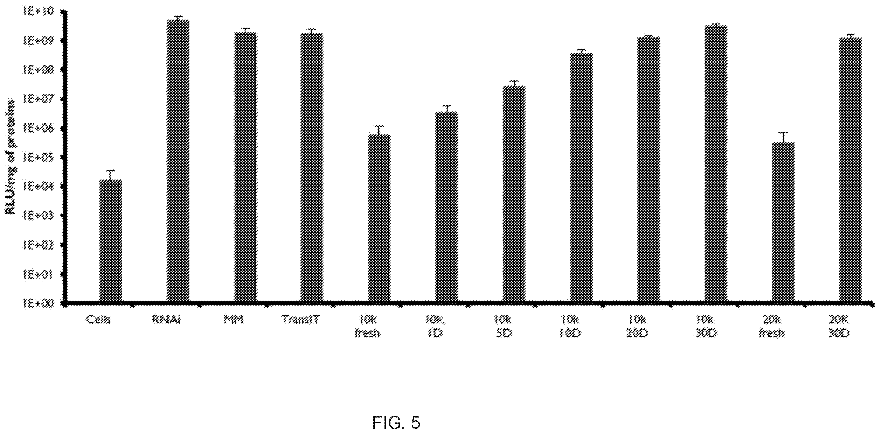

FIG. 5 is a bar graph showing transfection efficiency of aPACE in Daoy cells compared to non-activated PACE. Similar to the results in HEK293 cells, both 10 kDa PACE and 20 kDa PACE were more effective after activation up to 30 days. After 30 days of activation, both 10 kDa PACE and 20 kDa PACE displayed comparable transfection efficiency to TRANS-IT reagent.

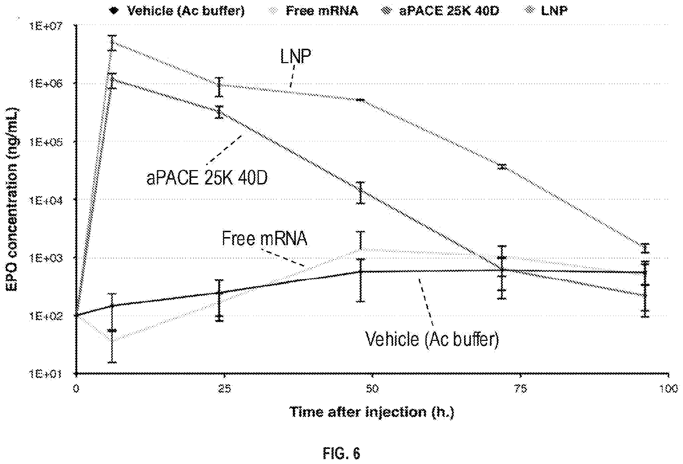

FIG. 6 is a line graph showing that aPACE effectively delivers erythropoietin (EPO) mRNA *in vivo.* A single dose of 20 µg EPO mRNA was injected into the tail vein of mice alone (Free mRNA), in combination with 25 kDa PACE activated for 40 days (aPACE 25K 40D), and in combination with Lipid Mix - LNP (Precision Nanosystems). EPO concentrations were measured in blood by enzyme-linked immunosorbent assay (ELISA) over time after transfection. Injection of free mRNA did not result in EPO production. However, both LNP and aPACE 25K 40D resulted in significant EPO production within 5 hours of injection and remaining above baseline for up to 50 hours post-injection.

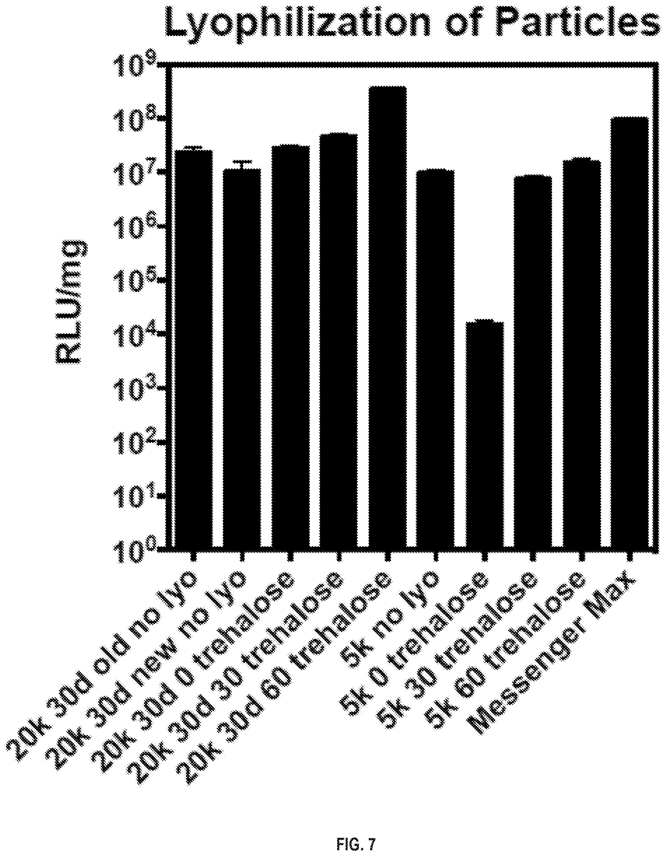

FIG. 7 is a bar graph demonstrating that aPACE formulations can by lyophilized for long-term storage without loss of activity. PACE (20-kDa) activated for 30 days was mixed with DNA to form aPACE/DNA polyplexes. The data indicate that use of the aPACE-nucleic acid complexes for *in vitro* transfection after lyophilization and storage for one month still results in effective activity.

### DETAILED DESCRIPTION

It is to be understood that the present disclosure is not limited to the particular embodiments of the disclosure described below, as variations of the particular embodiments may be made and still fall within the scope of the appended claims. Instead, the scope of the present disclosure will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this disclosure belongs.

The term "terpolymer" as used herein refers to a copolymer comprising three distinct monomers. In an embodiment, the terpolymer can consist of three distinct monomers.

The term "polyplex" as used herein refers to polymeric micro- and/or nanoparticles or micelles having encapsulated therein, dispersed within, and/or associated with the surface of, one or more polynucleotides.

As generally used herein "pharmaceutically acceptable" refers to those compounds, materials, compositions and/or dosage forms that are, within the scope of sound medical judgment, suitable for use in contact with the tissues, organs and/or bodily fluids of human beings and animals without excessive toxicity, irritation, allergic response or other problems or complications commensurate with a reasonable benefit/risk ratio.

The terms "subject," "individual" and "patient" refer to any individual who is the target of treatment using the disclosed compositions. The subject can be a vertebrate, for example, a mammal. Thus, the subject can be a human. The subjects can be symptomatic or asymptomatic. The term does not denote a particular age or sex. Thus, adult and newborn subjects, whether male or female, are intended to be included. A subject can include a control subject or a test subject.

The term "biocompatible," as used herein, refers to one or more materials that are neither themselves toxic to the host (*e*.*g*., an animal or human), nor degrade (if the material degrades) at a rate that produces monomeric or oligomeric subunits or other byproducts at toxic concentrations in the host.

The term "biodegradable" as used herein means that the materials degrade or break down into their component subunits, or digestion, *e*.*g*., by a biochemical process, of the material into smaller (*e*.*g*., non-polymeric) subunits.

The term "microspheres" is art-recognized and includes substantially spherical colloidal structures, *e*.*g*., formed from biocompatible polymers such as subject compositions, having a size ranging from about one or greater up to about 1000 microns. In general, "microcapsules," also an art-recognized term, are distinguished from microspheres, because microcapsules are generally covered by a substance of some type, such as a polymeric formulation. The term "microparticles" is also art-recognized, and includes microspheres and microcapsules, as well as structures that may not be readily placed into either of the above two categories, all with dimensions on average of less than about 1000 microns. A microparticle may be spherical or non-spherical and may have any regular or irregular shape. If the structures are less than about one micron in diameter, then the corresponding art-recognized terms "nanosphere," "nanocapsule" and "nanoparticle" may be utilized. In certain embodiments, the nanospheres, nanocapsules and nanoparticles have an average diameter of about 500 nm, 200 nm, 100 nm, 50 nm, 10 nm or 1 nm. In some embodiments, the average diameter of the particles is from about 200 nm to about 600 nm, *e.g.,* from about 200 nm to about 500 nm. Microparticles can be used, for example, for gene therapy, particularly for vaccinations, drug delivery, including macromolecular drug therapies, and enzyme replacement therapies.

A composition comprising microparticles or nanoparticles can include particles of a range of particle sizes. In certain embodiments, the particle size distribution may be uniform, *e*.*g*., within less than about a 20% standard deviation of the mean volume diameter, and in other embodiments, still more uniform, e.g., within about 10%, 8%, 5%, 3% or 2% of the median volume diameter.

The term "particle" as used herein refers to any particle formed of, having attached thereon or thereto, or incorporating a therapeutic, diagnostic or prophylactic agent, optionally including one or more polymers, liposomes, micelles, or other structural material. A particle may be spherical or non-spherical. A particle may be used, for example, for diagnosing a disease or condition, treating a disease or condition, or preventing a disease or condition.

The phrases "parenteral administration" and "administered parenterally" are art-recognized terms, and include modes of administration other than enteral and topical administration, such as, for example, injections, and include, without limitation, intravenous, intramuscular, intrapleural, intravascular, intrapericardial, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrastemal injection and infusion.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

The term construct refers to a recombinant genetic molecule having one or more isolated polynucleotide sequences.

A "transgenic organism" as used herein, is any organism, in which one or more of the cells of the organism contains heterologous nucleic acid introduced by way of human intervention, such as by transgenic techniques known in the art. Suitable transgenic organisms include, but are not limited to, bacteria, cyanobacteria, fungi, plants and animals. The formulations described herein, e.g., nucleic acids formulated in polymers described herein, can be introduced into the host by methods known in the art, for example infection, transfection, transformation or transconjugation. Techniques for transferring DNA into such organisms are known and provided in references such as Sambrook, et al. (2000) Molecular Cloning: A Laboratory Manual, 3rd ed., vol. 1-3, Cold Spring Harbor Press, Plainview N.Y.

As used herein, the term "eukaryote" or "eukaryotic" refers to organisms or cells or tissues derived therefrom belonging to the phylogenetic domain Eukarya such as animals (*e*.*g*., mammals, insects, reptiles, and birds), ciliates, plants (*e*.*g*., monocots, dicots, and algae), fungi, yeasts, flagellates, microsporidia and protists.

As used herein, the term "non-eukaryotic organism" refers to organisms includingorganisms of the Eubacteria phylogenetic domain, such as *Escherichia coli, Thermus thermophilus, and Bacillus stearothermophilus,* or organisms of the Archaea phylogenetic domain such as, *Methanocaldococcus jannaschii, Methanothermobacter thermautotrophicus,* Halobacterium such as *Haloferax volcanii* and Halobacterium species NRC-1, *Archaeoglobus fulgidus, Pyrococcus furiosus, Pyrococcus horikoshii,* and *Aeuropyrum pernix.*

The term "gene" refers to a DNA sequence that encodes through its template or messenger RNA a sequence of amino acids characteristic of a gene product, *e*.*g*., a specific peptide, polypeptide or protein. The term "gene" also refers to a DNA sequence that encodes an RNA product. The term gene as used herein with reference to genomic DNA includes intervening, non-coding regions as well as regulatory regions and can include 5' and 3' ends.

A "gene product" is a biopolymeric product that is expressed or produced by a gene. A gene product may be, for example, an unspliced RNA, an mRNA, a splice variant mRNA, a polypeptide, a post-translationally modified polypeptide, a splice variant polypeptide etc. Also encompassed by this term are biopolymeric products that are made using an RNA gene product as a template (*e*.*g*., cDNA of the RNA). A gene product may be made enzymatically, recombinantly, chemically, or within a cell to which the gene is native. In some embodiments, if the gene product is proteinaceous, it exhibits a biological activity. In some embodiments, if the gene product is a nucleic acid, it can be translated into a proteinaceous gene product that exhibits a biological activity.

The term polypeptide includes proteins and fragments thereof. The polypeptides can be "exogenous," meaning that they are "heterologous" (*i*.*e*., foreign to the host cell being utilized), such as human polypeptide produced by a bacterial cell. Polypeptides are disclosed herein as amino acid residue sequences. Those sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gin, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term "heterologous" refers to elements occurring where they are not normally found. For example, a promoter may be linked to a heterologous nucleic acid sequence, *e*.*g*., a sequence that is not normally found operably linked to the promoter. When used herein to describe a promoter element, heterologous means a promoter element that differs from that normally found in the native promoter, either in sequence, species or number. For example, a heterologous control element in a promoter sequence may be a control/regulatory element of a different promoter added to enhance promoter control, or an additional control element of the same promoter. The term "heterologous" thus can also encompass "exogenous" and "non-native" elements.

"Variant" refers to a polypeptide or polynucleotide that differs from a reference polypeptide or polynucleotide, but retains essential properties. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (*e*.*g*., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

Modifications and changes can be made in the structure of the polypeptides that do not significantly alter the characteristics of the polypeptide (*e*.*g*., a conservative amino acid substitution). For example, certain amino acids can be substituted for other amino acids in a sequence without appreciable loss of activity. Because it is the interactive capacity and nature of a polypeptide that defines that polypeptide's biological functional activity, certain amino acid sequence substitutions can be made in a polypeptide sequence and nevertheless obtain a polypeptide with like properties.

Amino acid substitutions are generally based on the relative similarity of the amino acid side-chain substituents, for example, their hydrophobicity, hydrophilicity, charge, size. Exemplary substitutions that take various of the foregoing characteristics into consideration are known to those of skill in the art and include (original residue: exemplary substitution): (Ala: Gly, Ser), (Arg: Lys), (Asn: Gln, His), (Asp: Glu, Cys, Ser), (Gln: Asn), (Glu: Asp), (Gly: Ala), (His: Asn, Gin), (Ile: Leu, Val), (Leu: Ile, Val), (Lys: Arg), (Met: Leu, Tyr), (Ser: Thr), (Thr: Ser), (Trp: Tyr), (Tyr: Trp, Phe), and (Val: Ile, Leu). Embodiments of this disclosure thus contemplate functional or biological equivalents of a polypeptide as set forth above. In particular, embodiments of the polypeptides can include variants having about 50%, 60%, 70%, 80%, 90%, 95%, 96%, 97%, 98%, 99%, or more sequence identity to the polypeptide of interest.

The term "percent identity" is defined in reference to a polynucleotide or amino acid sequence, as the percentage of nucleotides or amino acids in a candidate sequence that are identical with the nucleotides or amino acids in a reference nucleic acid sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

The term "isolated" is meant to describe a compound of interest (*e*.*g*., nucleic acids) that is in an environment different from that in which the compound naturally occurs, *e*.*g*., separated from its natural milieu such as by concentrating a peptide to a concentration at which it is not found in nature. "Isolated" is meant to include compounds that are within samples that are substantially enriched for the compound of interest and/or in which the compound of interest is partially or substantially purified. Isolated nucleic acids are at least about 60% free, about 75% free or about 90% free from other associated components.

The term "vector" refers to a replicon, such as a plasmid, phage, virus, modified virus or cosmid, into which another DNA segment may be inserted so as to bring about the replication of the inserted segment. The vectors can be expression vectors. The term "expression vector" refers to a vector that includes one or more expression control sequences.

"Transformed," "transgenic," "transfected" and "recombinant" refer to a host organism into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, organisms, subjects or plants are understood to encompass not only the end product of a transformation process, but also transgenic progeny thereof. A "non-transformed," "non-transgenic" or "non--recombinant" host refers to an otherwise "wild-type" organism, *e*.*g*., a cell, bacterium, organism, subject or plant, that does not contain the heterologous nucleic acid molecule.

### I. Polymers

Polymers comprising one or more backbone ester(s) that have been activated by temperature-controlled hydrolysis, thereby exposing one or more activated end group(s), are disclosed herein. The one or more activated end group(s) can be, for example, hydroxyl or carboxylic acid end groups. The activated polymers range in size, for example, from about 5 to 25 kDa, preferably about 10 kDa, in size. As used herein, the term "about" is meant to minor variations within acceptable parameters. For the sake of clarity, "about" refers to ±10% of a given value.

As claimed, PACE polymers that have been activated by, for example, temperature-controlled hydrolysis polymers.

The PACE polymer to be activated as defined in claim 1 is poly(amine co ester) synthesized by a method comprising combining 15 pentadecanolide (PDL), diethanolamine, and a diester/diacid selected from either diethyl sebacate (DES) or sebacic acid (SBA).
which is encompassed by the general formula (not claimed): 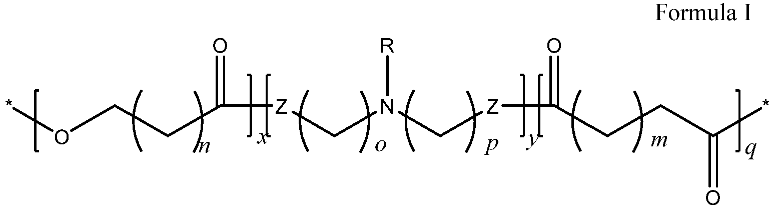 wherein *n* is an integer from 1-30; m, *o* and *p* are independently an integer from 1-20; *x, y* and *q* are independently integers from 1-1000; and Z is O or NR', wherein R' is hydrogen, substituted or unsubstituted alkyl, or substituted or unsubstituted aryl. R is alkyl (*e*.*g*., methyl, *t*-butyl or ethyl), alkoxy (*e*.*g*., or hydroxyethyl) or aryl. The polymer can be prepared from one or more lactones, one or more amine-dials or triamines, and one or more diacids or diesters. In those embodiments where two or more different lactone, diacid or diester, and/or triamine or amine-dial monomers are used, then the values of *n, o, p* and/or *m* can be the same or different.

In some embodiments (not claimed), Z is O, *n* is an integer from 1-16, such 4, 10, 13 or 14; *m* is an integer from 1-10, such as 4, 5, 6, 7 or 8; and/or R is alkyl, such a methyl, ethyl, *n*-propyl, isopropyl, *n*-butyl or *t*-butyl, or aryl, such as phenyl.

As claimed, *n* is 14 (*e*.*g*., pentadecalactone, PDL), *m* is 7 (*e*.*g*., diethylsebacate, DES).

As used herein, "alkyl" means a noncyclic straight chain or branched, unsaturated or saturated hydrocarbon such as those containing from 1 to 10 carbon atoms, while the term "lower alkyl" or "C1-6 alkyl" has the same meaning as alkyl but contains from 1 to 6 carbon atoms. The term "higher alkyl" has the same meaning as alkyl but contains from 7 to 10 carbon atoms. Representative saturated straight chain alkyls include, for example, methyl, ethyl, n-propyl, n-butyl, n-pentyl, n-hexyl, n-septyl, n-octyl, n-nonyl, and the like; while saturated branched alkyls include isopropyl, sec-butyl, isobutyl, tert-butyl, isopentyl, and the like. Unsaturated alkyls contain at least one double or triple bond between adjacent carbon atoms (referred to as an "alkenyl" or "alkynyl," respectively). Representative straight chain and branched alkenyls include, for example, ethylenyl, propylenyl, 1-butenyl, 2-butenyl, isobutylenyl, 1-pentenyl, 2-pentenyl, 3 -methyl- 1-butenyl, 2-methyl-2-butenyl, 2,3- dimethyl-2-butenyl, and the like; while representative straight chain and branched alkynyls include acetylenyl, propynyl, 1-butynyl, 2-butynyl, 1-pentynyl, 2-pentynyl, 3-methyl-1-butynyl, and the like.

"Alkoxy" refers to an alkyl group as defined above with the indicated number of carbon atoms attached through an oxygen bridge. Examples of alkoxy include, but are not limited to, methoxy, ethoxy, n-propoxy, i-propoxy, n-butoxy, s-butoxy, t-butoxy, n- pentoxy, and s-pentoxy.

"Aryl" means an aromatic carbocyclic monocyclic or polycyclic ring such as phenyl or naphthyl.

The activated PACE polymers described herein compriseone or more activated end group(s) selected from the group consisting of hydroxyl or carboxylic acid end groups.

The polymer can be biocompatible. In some embodiments, the polymer is biocompatible and biodegradable. The therapeutic agent(s), *e*.*g*., nucleic acid(s), encapsulated by and/or associated with the particles can be released through different mechanisms, including diffusion and degradation of the polymeric matrix. The rate of release can be controlled by varying the monomer composition of the polymer and thus the rate of degradation. If, for example, simple hydrolysis is the primary mechanism of degradation, increasing the hydrophobicity of the polymer may slow the rate of degradation and therefore increase the time period of release. In all case, the polymer composition is selected such that an effective amount of nucleic acid(s) is released to achieve the desired purpose/outcome.

### II. Microparticles Formed from the Polymers

The polymers described herein can be used to prepare microparticles and/or nanoparticles having encapsulated therein one or more therapeutic, diagnostic or prophylactic agents. The agent can be encapsulated within the particle, dispersed within the polymer matrix that forms the particle, covalently or non-covalently associated with the surface of the particle or combinations thereof.

The agent to be encapsulated and delivered can be a small molecule agent (e.g., non-polymeric agent having a molecular weight less than about 2,000, 1500, 1,000, 750 or 500 Da) or a macromolecule (*e*.*g*., an oligomer or polymer) such as, for example, protein, enzyme, peptide, nucleic acid, antibody, siRNA, mRNA, etc. Suitable small molecule active agents include organic, inorganic, and/or organometallic compounds. The particles can be used for *in vivo* and/or *in vitro* delivery of the agent.

Exemplary therapeutic agents that can be incorporated into the particles include, but are not limited to tumor antigens, immune suppressors or activators (*e*.*g*., inhibitors of adaptive or passive immune responses, complement inhibitors or activators, etc.), CD4⁺ T-cell epitopes, cytokines, chemotherapeutic agents, radionuclides, small molecule signal transduction inhibitors, photothermal antennas, monoclonal antibodies, immunologic danger signaling molecules, other immunotherapeutics, enzymes, antibiotics, antivirals (especially protease inhibitors alone or in combination with nucleosides for treatment of HIV or Hepatitis B or C), anti-parasitics (helminths, protozoans), growth factors, growth inhibitors, hormones, hormone antagonists, antibodies and bioactive fragments thereof (including humanized, single chain, and chimeric antibodies), antigen and vaccine formulations (including adjuvants), peptide drugs, anti-inflammatories, immunomodulators (including ligands that bind to Toll-Like Receptors to activate the innate immune system, molecules that mobilize and optimize the adaptive immune system, molecules that activate or up-regulate the action of cytotoxic T lymphocytes, natural killer cells and helper T-cells, and molecules that deactivate or down-regulate suppressor or regulatory T-cells ), agents that promote uptake of the particles into cells (including dendritic cells and other antigen-presenting cells), nutraceuticals such as vitamins, and oligonucleotide drugs (including DNA, RNA, mRNA, antisense, aptamers, small interfering RNAs, ribozymes, external guide sequences for ribonuclease P, and triplex forming agents).

Representative anti-cancer agents include, but are not limited to, alkylating agents (such as cisplatin, carboplatin, oxaliplatin, mechlorethamine, cyclophosphamide, chlorambucil, dacarbazine, lomustine, carmustine, procarbazine, chlorambucil and ifosfamide ), antimetabolites (such as fluorouracil (5-FU), gemcitabine, methotrexate, cytosine arabinoside, fludarabine, and floxuridine), antimitotics (including taxanes such as paclitaxel and docetaxel and vinca alkaloids such as vincristine, vinblastine, vinorelbine, and vindesine), anthracyclines (including doxorubicin, daunorubicin, valrubicin, idarubicin, and epirubicin, as well as actinomycins such as actinomycin D), cytotoxic antibiotics (including mitomycin, plicamycin, and bleomycin), topoisomerase inhibitors (including camptothecins such as camptothecin, irinotecan, and topotecan as well as derivatives of epipodophyllotoxins such as amsacrine, etoposide, etoposide phosphate, and teniposide), antibodies to vascular endothelial growth factor (VEGF) such as bevacizumab (AVASTIN^{®}), other anti-VEGF compounds; thalidomide (THALOMID^{®}) and derivatives thereof such as lenalidomide (REVLIMID^{®}); endostatin; angiostatin; receptor tyrosine kinase (RTK) inhibitors such as sunitinib (SUTENT^{®}); tyrosine kinase inhibitors such as sorafenib (Nexavar^{®}), erlotinib (Tarceva^{®}), pazopanib, axitinib, and lapatinib; transforming growth factor-α or transforming growth factor-β inhibitors, and antibodies to the epidermal growth factor receptor such as panitumumab (VECTIBIX^{®}) and cetuximab (ERBITUX^{®}).

Exemplary immunomodulatory agents include, antigens that inhibit or activate the complement response (*e*.*g*., classical pathway, alternative pathway and/or lectin pathway), cytokines, xanthines, interleukins, interferons, oligodeoxynucleotides, glucans, growth factors (*e*.*g*., TNF, CSF, GMCSF and G-CSF), hormones such as estrogens (diethylstilbestrol, estradiol), androgens (testosterone, HALOTESTIN^{®} (fluoxymesterone)), progestins (MEGACE^{®} (megestrol acetate), PROVERA^{®} (medroxyprogesterone acetate)), and corticosteroids (prednisone, dexamethasone, hydrocortisone).

Examples of immunological adjuvants that can be associated with the particles include, but are not limited to, TLR ligands, C-Type Lectin Receptor ligands, NOD-Like Receptor ligands, RLR ligands, and RAGE ligands. TLR ligands can include lipopolysaccharide (LPS) and derivatives thereof, as well as lipid A and derivatives there of includingmonophosphoryl lipid A (MPL), glycopyranosyl lipid A, PET-lipid A, and 3-O-desacyl-4'-monophosphoryl lipid A.

The particles may also include antigens and/or adjuvants (*e*.*g*., molecules enhancing an immune response). Peptide, protein, and DNA based vaccines may be used to induce immunity to various diseases or conditions. Cell-mediated immunity is needed to detect and destroy virus-infected cells. Most traditional vaccines (*e*.*g*., protein-based vaccines) can only induce humoral immunity. DNA-based vaccine represents a unique means to vaccinate against a virus or parasite because a DNA-based vaccine can induce both humoral and cell-mediated immunity. In addition, DNA based vaccines are potentially safer than traditional vaccines. DNA vaccines are relatively more stable and more cost-effective for manufacturing and storage. DNA vaccines consist of two major components- DNA carriers (or delivery vehicles) and DNAs encoding antigens. DNA carriers protect DNA from degradation, and can facilitate DNA entry to specific tissues or cells and expression at an efficient level.

Exemplary diagnostic agents include paramagnetic molecules, fluorescent compounds, magnetic molecules, and radionuclides, x-ray imaging agents, and contrast agents.

In some embodiments, particles produced using the methods described herein contain less than 80%, less than 75%, less than 70%, less than 60%, less than 50% by weight, less than 40% by weight, less than 30% by weight, less than 20% by weight, less than 15% by weight, less than 10% by weight, less than 5% by weight, less than 1 % by weight, less than 0.5% by weight, or less than 0.1 % by weight of the agent.

In some embodiments, the agent may be a mixture of pharmaceutically active agents. The percent loading is dependent on a variety of factors, including the agent to be encapsulated, the polymer used to prepare the particles, and/or the method used to prepare the particles.

### III. Compositions for Transfection of Polynucleotides

The gene delivery ability of polycationic polymers is due to multiple factors, including polymer molecular weight, hydrophobicity and charge density. Many synthetic polycationic materials have been tested as vectors for non-viral gene delivery, but almost all are ineffective due to their low efficiency or high toxicity. Most polycationic vehicles described previously exhibit high charge density, which has been considered a major requirement for effective DNA condensation. As a result, they are able to deliver genes with high efficiency *in vitro* but are limited for *in vivo* applications because of toxicity related to the excessive charge density.

High molecular weight polymers, particularly terpolymers, and methods of making them using, for example, enzyme-catalyzed copolymerization of a lactone with a dialkyl diester and an amino diol have been previously disclosed. These PACE terpolymers have a low charge density. In addition, their hydrophobicity can be varied by selecting a lactone co-monomer with specific ring size and by adjusting lactone content in the polymers. High molecular weight and increased hydrophobicity of the lactone-diester-amino dial terpolymers result in minimal toxicity. However, these polyplexes are unstable in serum, thus limiting their effectiveness.

Methods of chemical modification of PACE polymers that produce low molecular weight polymers, referred to as activated PACE (aPACE) are disclosed herein. These aPACE terpolymers vary between about 5 kDa and about 10 kDa in size and end in either -OH or -COOH. aPACE terpolymers can be used to efficiently and safely deliver polynucleotides, such as DNA or RNA, including mRNA, both *in vitro* and *in vivo.*

The aPACE terpolymers exhibit efficient gene delivery with reduced toxicity. The aPACE terpolymers can be significantly more efficient than commercially available non-viral vectors. The aPACE terpolymers described herein, for example, can be at least as efficient or more efficient than commercially available non-viral vectors such as TRANS-IT and LIPOFECTAMINE based on luciferase expression assay while exhibiting minimal to no toxicity at doses of up to 20 µg/mL. The aPACE terpolymer is generally non-toxic at concentrations suitable for both *in vitro* and *in vivo* transfection of nucleic acids. The aPACE terpolymers described herein, for example, cause less non-specific cell death compared to other approaches of cell transfection.

### IV. Methods of Making aPACE Polymers

Methods for the synthesis of an aPACE polymer can comprise for example, creating a PACE polymer by, for example, combining one or more lactones, one or more amine-diols or triamines, and one or more diacids or diesters in the presence of a catalyst under atmospheric pressure at about 90C for 24 hours, reducing the reaction pressure to about 1.6 mmHg and continuing the reaction at about 90C for an additional 8 to 72 hours. Activation of a PACE polymer, as formed according to the current invention as defined by the claims is accomplished by hydrolyzing the terpolymers produced for about 1 day to about 30 days or more, as defined in the claims.

In a general context (not claimed), the PACE polymers may be prepared as shown in Scheme 1: 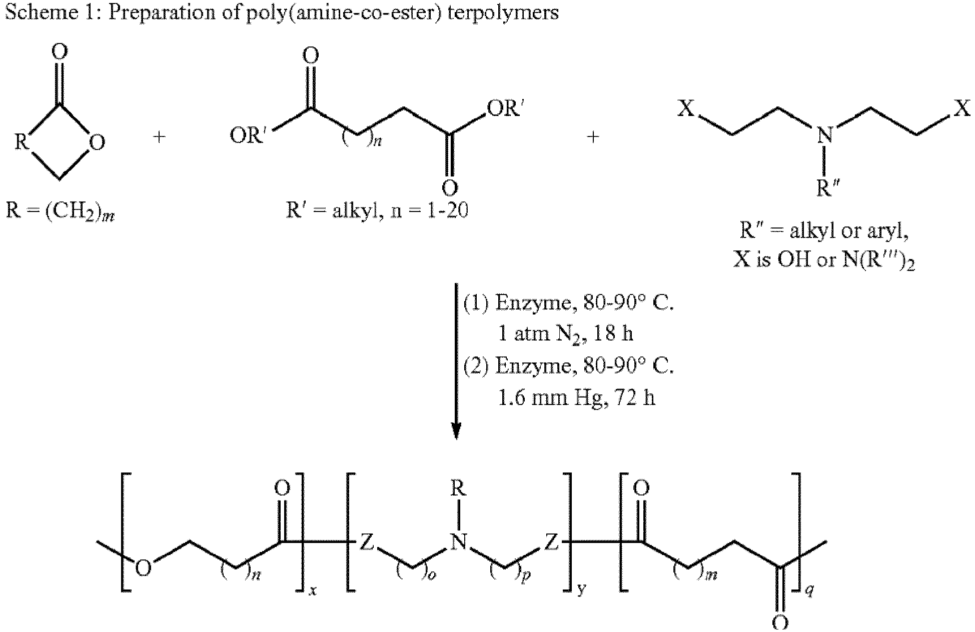 wherein n is an integer from 1-30; m, o and p are independently an integer from 1-20; and x, y and q are independently integers from 1-1000; Z is O or NR‴, and R‴ is hydrogen, substituted or unsubstituted alkyl, or substituted or unsubstituted aryl. The polymer can be prepared from one or more lactones, one or more amine-dials or triamines, and one or more diacids or diesters. In those embodiments where two or more different lactone, diacid or diester, and/or triamine or amine-dial monomers are used, the values of n, o, p and/or m can be the same or different.

The molar ratio of the monomers can vary, for example from about 10:90:90 to about 90:10:10. In some embodiments, the ratio is 10:90:90, 20:80:80, 40:60:60, 60:40:40 or 80:20:20. The weight average molecular weight, as determined by GPC using narrow polydispersity polystyrene standards, can vary for example from about 10,000 Daltons to about 50,000 Daltons.

In accordance with the method of claim 9, PACE polymers are synthesized from 15-pentadecanolide (PDL), diethyl sebacate (DES) / sebacic acid (SBA), and diethanolamine (*e*.*g*., N-methyl-diethanolamine (MDEA)) using an enzyme catalyst, such as *Candida antartica* lipase B (CALB), as illustrated in FIG. 1 (ratio of PDL:DES:MDEA = 1:9:9 for this example). The reaction can be catalyzed by other catalysts, *e*.*g*., metal catalysts.

Any PACE polymers can be activated by a temperature-controlled hydrolysis reaction for up to 30 days or more. The length of hydrolysis may vary depending on the molecular weight of the PACE polymer to be activated. Larger molecular weight polymers (*e*.*g*., about 20-25 kDa) are optimally hydrolyzed for longer periods of time, for example, for about 30 to 40 days. Smaller molecular weight polymers (*e*.*g*., about 5-7 kDa) are optimally hydrolyzed for shorter periods of time, for example, for about 4 to 10 days.

The PACE polymers are hydrolyzed at a temperature from about 30C to 42C, or any in the range of up to about 100C. The PACE polymers can be hydrolyzed at a temperature from about 35C to 40C, *e*.*g*., about 37C.

According to the invention the PACE polymers are hydrolyzed at about 1 atm. Higher pressures accelerate the process (*e*.*g*., pressures from about 1 to about 100 atm). The rate for the process would be determined by one of skill in the art for the specific formulations being made.

The average molecular weight of the resulting activated PACE polymers can vary from about 5 kDa to about 25 kDa and is defined as between 5kDa to 10kDa in the claims. The end-groups for the aPACE polymers can be independently a carboxyl, or a hydroxyl

### V. Therapeutic (not claimed), Prophylactic and Diagnostic Use of aPACE Polymers

The polymers described herein can form various polymer compositions, which are useful for preparing a variety of biodegradable medical devices and for drug delivery. Devices prepared from the aPACE polymers described herein can be used for a wide range of medical applications. Examples of such applications include, but are not limited to, controlled release of therapeutic, prophylactic or diagnostic agents; drug delivery; tissue engineering scaffolds; cell encapsulation; targeted delivery; biocompatible coatings; biocompatible implants; guided tissue regeneration; wound dressings; orthopedic devices; prosthetics and bone cements (including adhesives and/or structural fillers); and diagnostics.

The polymers described herein can be used to encapsulate, be mixed with, or be ionically or covalently coupled to any of a variety of therapeutic, prophylactic or diagnostic agents. A wide variety of biologically active materials can be encapsulated or incorporated, either for delivery to a site by the polymer, or to impart properties to the polymer, such as bioadhesion, cell attachment, enhancement of cell growth, inhibition of bacterial growth, and prevention of clot formation.

Examples of suitable therapeutic and prophylactic agents include synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules that bind to complementary DNA to inhibit transcription, siRNA, mRNA and ribozymes. Compounds with a wide range of molecular weight can be encapsulated, for example, between 100 and 500,000 grams or more per mole. Examples of suitable materials include proteins such as antibodies, receptor ligands, and enzymes, peptides such as adhesion peptides, saccharides and polysaccharides, synthetic organic or inorganic drugs, and nucleic acids. Examples of materials that can be encapsulated include enzymes, blood clotting factors, inhibitors or clot dissolving agents such as streptokinase and tissue plasminogen activator; antigens for immunization; hormones and growth factors; polysaccharides such as heparin; oligonucleotides such as antisense oligonucleotides and ribozymes and retroviral vectors for use in gene therapy. The polymer can also be used to encapsulate cells and tissues. Representative diagnostic agents are agents detectable, for example, by x-ray, fluorescence, magnetic resonance imaging, radioactivity, ultrasound, computer tomagraphy (CT) and positron emission tomagraphy (PET). Ultrasound diagnostic agents are typically a gas such as air, oxygen or perfluorocarbons.

### VI. Polynucleotides

The aPACE terpolymers described herein can be used, for example, to transfect cells with nucleic acids. Accordingly, polyplexes including aPACE terpolymers and one or more polynucleotides are also disclosed.

The polynucleotide can encode one or more proteins, functional nucleic acids, or combinations thereof. The polynucleotide can be monocistronic or polycistronic. In some embodiments, polynucleotide is multigenic.

In some embodiments, the polynucleotide is transfected into the cell and remains extrachromosomal. In some embodiments, the polynucleotide is introduced into a host cell and is integrated into the host cell's genome. The compositions and formulations can be used, for example, in methods of gene therapy (not claimed). Methods of gene therapy can include the introduction into the cell of a polynucleotide that alters the genotype of the cell. Introduction of the polynucleotide can correct, replace or otherwise alter the endogenous gene via genetic recombination. Methods can include introduction of an entire replacement copy of a defective gene, a heterologous gene, or a small nucleic acid molecule such as an oligonucleotide. A corrective gene, for example, can be introduced into a non-specific location within a host's genome.

In some embodiments, the polynucleotide is incorporated into or part of a vector. Methods to construct expression vectors containing genetic sequences and appropriate transcriptional and translational control elements are known in the art. These methods include *in vitro* recombinant DNA techniques, synthetic techniques, and *in vivo* genetic recombination. Expression vectors generally contain regulatory sequences and necessary elements for the translation and/or transcription of the inserted coding sequence, which can be, for example, the polynucleotide of interest. The coding sequence can be operably linked to a promoter and/or enhancer to help control the expression of the desired gene product. Promoters used in biotechnology are of different types according to the intended type of control of gene expression. They can be generally divided into constitutive promoters, tissue-specific or development-stage-specific promoters, inducible promoters, and synthetic promoters.

The polynucleotide of interest can be operably linked to a promoter or other regulatory elements, for example. Thus, the polynucleotide can be a vector such as an expression vector. The engineering of polynucleotides for expression in a prokaryotic or eukaryotic system may be performed by techniques generally known to those of skill in recombinant expression. An expression vector typically comprises one of the disclosed compositions under the control of one or more promoters. To bring a coding sequence "under the control of" a promoter, one positions the 5' end of the translational initiation site of the reading frame generally between about 1 and 50 nucleotides "downstream" of (*i.e.,* 3' of) the chosen promoter. The "upstream" promoter stimulates transcription of the inserted DNA and promotes expression of the encoded recombinant protein. This is the meaning of "recombinant expression" in the context used here.

Many techniques are available to construct expression vectors containing the appropriate nucleic acids and transcriptional/translational control sequences to achieve protein or peptide expression in a variety of host-expression systems. Cell types available for expression include, but are not limited to, bacteria, such as, for example, *E. coli* and *B. subtilis* transformed with recombinant phage DNA, plasmid DNA or cosmid DNA expression vectors. It will be appreciated that any of these vectors may be packaged and delivered using the disclosed polymers.

Expression vectors for use in mammalian cells ordinarily include an origin of replication (as necessary), a promoter located in front of the gene to be expressed, along with any necessary ribosome binding sites, RNA splice sites, polyadenylation site, and transcriptional terminator sequences. The origin of replication may be provided either by construction of the vector to include an exogenous origin, such as may be derived from SV40 or other viral (*e*.*g*., polyoma, adeno, VSV, BPV) source, or may be provided by the host cell chromosomal replication mechanism. If the vector is integrated into the host cell chromosome, the latter is often sufficient.

The promoters may be derived from the genome of mammalian cells (*e*.*g*., metallothionein promoter) or from mammalian viruses (*e*.*g*., the adenovirus late promoter; the vaccinia virus 7.5K promoter). It is also possible, and may be desirable, to utilize promoter or control sequences normally associated with the desired gene sequence, provided such control sequences are compatible with the host cell systems.

A number of viral-based expression systems may be utilized, for example, commonly used promoters are derived from polyoma, adenovirus 2, cytomegalovirus and SV40. The early and late promoters of SV40 virus are useful because both are obtained easily from the virus as a fragment that also contains the SV40 viral origin of replication. Smaller or larger SV40 fragments may also be used, provided there is included the approximately 250 bp sequence extending from the *Hind*III site toward the *Bgl*I site located in the viral origin of replication.

In cases where an adenovirus is used as an expression vector, the coding sequences may be ligated to an adenovirus transcription/translation control complex, *e*.*g*., the late promoter and tripartite leader sequence. This chimeric gene may then be inserted into, for example, an adenovirus genome by *in vitro* or *in vivo* recombination. Insertion in a non-essential region of the viral genome (*e*.*g*., region E1 or E3) results in a recombinant virus that is viable and capable of expressing proteins in infected hosts.

Specific initiation signals may also be required for efficient translation of the disclosed compositions. These signals include the ATG initiation codon and adjacent sequences. Exogenous translational control signals, including the ATG initiation codon, may additionally need to be provided. One of ordinary skill in the art would readily be capable of determining this need and providing the necessary signals. It is known that the initiation codon must be in-frame (or in-phase) with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements or transcription terminators.

In eukaryotic expression, one will also typically desire to incorporate into the transcriptional unit an appropriate polyadenylation site if one was not contained within the original cloned segment. The poly-A addition site is typically placed about 30 to 2000 nucleotides "downstream" of the termination site of the protein at a position prior to transcription termination.

For long-term, high-yield production of recombinant proteins, stable expression is preferred. Cell lines that stably express constructs encoding proteins, for example, may be engineered. Rather than using expression vectors that contain viral origins of replication, host cells can be transformed with vectors controlled by appropriate expression control elements (*e*.*g*., promoter, enhancer, sequences, transcription terminators, polyadenylation sites, etc.), and a selectable marker. Following the introduction of foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci, which in turn can be cloned and expanded into cell lines.

### VII. Polypeptide of Interest

The polynucleotide can encode one or more polypeptides of interest. The polypeptide can be any polypeptide. The polypeptide encoded by the polynucleotide, for example, can be a polypeptide that provides a therapeutic or prophylactic effect to an organism or that can be used to diagnose a disease or disorder in an organism. For treatment of cancer, autoimmune disorders, parasitic, viral, bacterial, fungal or other infections, the polynucleotide(s) to be expressed, for example, may encode a polypeptide that functions as a ligand or receptor for cells of the immune system, or can function to stimulate or inhibit the immune system of an organism.

In some embodiments, the polynucleotide supplements or replaces a polynucleotide that is defective in the organism.

In some embodiments, the polynucleotide includes a selectable marker, for example, a selectable marker that is effective in a eukaryotic cell, such as a drug resistance selection marker. This selectable marker gene can encode a factor necessary for the survival or growth of transformed host cells grown in a selective culture medium. Typical selection genes encode proteins that confer resistance to antibiotics or other toxins, *e*.*g*., ampicillin, neomycin, methotrexate, kanamycin, gentamycin, Zeocin or tetracycline, complement auxotrophic deficiencies, or supply critical nutrients withheld from the media.

In some embodiments, the polynucleotide includes a reporter gene. Reporter genes are typically genes that are not present or expressed in the host cell. The reporter gene typically encodes a protein that provides for some phenotypic change or enzymatic property, e.g., glucuronidase (GUS) and green fluorescent protein (GFP).

### VIII. Functional Nucleic Acids

The polynucleotide can be, or can encode a functional nucleic acid. Functional nucleic acids are nucleic acid molecules that have a specific function, such as binding a target molecule or catalyzing a specific reaction. Functional nucleic acid molecules can be divided into the following non-limiting categories: antisense molecules, siRNA, miRNA, aptamers, ribozymes, triplex forming molecules, RNAi, and external guide sequences. The functional nucleic acid molecules can act as effectors, inhibitors, modulators and stimulators of a specific activity possessed by a target molecule, or the functional nucleic acid molecules can possess a de novo activity independent of any other molecules.

Functional nucleic acid molecules can interact with any macromolecule, such as, for example, DNA, RNA, polypeptides or carbohydrate chains. Thus, functional nucleic acids can interact with the mRNA or the genomic DNA of a target polypeptide or they can interact with the polypeptide itself. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place.

Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively, the antisense molecule is designed to interrupt a processing function that normally would take place on the target molecule, such as transcription or replication. Antisense molecules can be designed based on the sequence of the target molecule. There are numerous methods for optimization of antisense efficiency by finding the most accessible regions of the target molecule. Exemplary methods include *in vitro* selection experiments and DNA modification studies using DMS and DEPC. It is preferred that antisense molecules bind the target molecule with a dissociation constant (K*_{d}*) less than or equal to 10⁻⁶, 10⁻⁸, 10⁻¹⁰ or 10⁻¹².

Aptamers are molecules that interact with a target molecule, preferably in a specific way. Typically, aptamers are small nucleic acids ranging from 15-50 bases in length that fold into defined secondary and tertiary structures, such as stem-loops or G-quartets. Aptamers can bind small molecules, such as ATP and theophiline, as well as large molecules, such as reverse transcriptase and thrombin. Aptamers can bind very tightly with K*_{d}*'s from the target molecule of less than 10⁻¹² M. The aptamers can bind the target molecule, for example, with a K*_{d}* less than 10⁻⁶, 10⁻⁸, 10⁻¹⁰ or 10⁻¹². Aptamers can bind the target molecule with a very high degree of specificity. For example, aptamers have been isolated that have greater than a 10,000-fold difference in binding affinities between the target molecule and another molecule that differ at only a single position on the molecule. The aptamers can have, for example, a K*_{d}* with the target molecule at least 10, 100, 1000, 10,000, or 100,000 fold lower than the K*_{d}* with a background binding molecule. When doing the comparison for a molecule such as a polypeptide, the background molecule is typically a different polypeptide.

Ribozymes are nucleic acid molecules that are capable of catalyzing a chemical reaction, either intramolecularly or intermolecularly. There are a number of different types of ribozymes that catalyze nuclease or nucleic acid polymerase type reactions that are based on ribozymes found in natural systems, such as, for example, hammerhead ribozymes. There are also a number of ribozymes that are not found in natural systems, but have been engineered to catalyze specific reactions *de novo.* Ribozymes can cleave RNA or DNA substrates for example. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrate's sequence.

Triplex forming functional nucleic acid molecules are molecules that can interact with either double-stranded or single-stranded nucleic acid. A structure called a triplex is formed where there are three strands of DNA forming a complex dependent on both Watson-Crick and Hoogsteen base-pairing. Triplex molecules can bind target regions, for example, with high affinity and specificity. Triplex-forming molecules can bind a target molecule, for example, with a K*_{d}* less than 10⁻⁶, 10⁻⁸, 10⁻¹⁰ or 10⁻¹².

External guide sequences (EGSs) are molecules that bind a target nucleic acid molecule forming a complex, which is recognized by RNAseP, which then cleaves the target molecule. EGSs can be designed to specifically target a RNA molecule of choice. RNAseP aids in processing transfer RNA (tRNA) within a cell. Bacterial RNAseP can be recruited to cleave virtually any RNA sequence by using an EGS that causes the target RNA:EGS complex to mimic the natural tRNA substrate. Similarly, eukaryotic EGS/RNAseP-directed cleavage of RNA can be utilized to cleave desired targets within eukaryotic cells. Representative examples of how to make and use EGS molecules to facilitate cleavage of a variety of different target molecules are known in the art.

Gene expression can also be effectively silenced in a highly specific manner through RNA interference (RNAi). This silencing was originally observed with the addition of double-stranded RNA (dsRNA). Once dsRNA enters a cell, it is cleaved by an RNaselll-like enzyme, Dicer, into double-stranded small interfering RNAs (siRNA), which are 21-23 nucleotides in length and contain two nucleotide overhangs at the 3' ends. In an ATP-dependent step, the siRNAs become integrated into a multi-subunit protein complex, commonly known as the RNAi induced silencing complex (RISC), which guides the siRNAs to the target RNA sequence. At some point the siRNA duplex unwinds, and the antisense strand remains bound to RISC, directing degradation of the complementary mRNA sequence by a combination of endo- and exonucleases. In one example, siRNA triggers the specific degradation of homologous RNA molecules, such as mRNAs, within the region of sequence identity between both the siRNA and the target RNA. However, the effect of iRNA or siRNA or their use is not limited to any type of mechanism.

WO 02/44321, herein incorporated by reference for the method of making these siRNAs, discloses siRNAs capable of sequence-specific degradation of target mRNAs when base-paired with 3' overhanging ends. siRNA can be chemically synthesized, synthesized in vitro or can be generated as the result of short double-stranded hairpin-like RNAs (shRNAs) that are processed into siRNAs inside the cell. Synthetic siRNAs are generally designed using algorithms and a conventional DNA/RNA synthesizer.

The production of siRNA from a vector is more commonly done through the transcription of shRNAs. Kits for the production of vectors comprising shRNA are available, such as, for example, GENESUPPRESSOR^{™} construction kits and Invitrogen's BLOCK-IT^{™} inducible RNAi plasmid and lentivirus vectors.

### IX. Composition of the Polynucleotides

The polynucleotide can be DNA or RNA nucleotides that typically include a heterocyclic base (nucleic acid base), a sugar moiety attached to the heterocyclic base, and a phosphate moiety that esterifies a hydroxyl function of the sugar moiety. The principal naturally occurring nucleotides comprise uracil, thymine, cytosine, adenine and guanine as the heterocyclic bases, and ribose or deoxyribose sugar linked by phosphodiester bonds. The polynucleotides can also include non-naturally occurring bases or bases that are otherwise chemically modified.

The polynucleotide can be composed of nucleotide analogs that have been chemically modified to improve stability, half-life, or specificity or affinity for a target sequence, relative to a DNA or RNA counterpart. The chemical modifications include chemical modification of nucleobases, sugar moieties, nucleotide linkages, or combinations thereof. As used herein "modified nucleotide" or "chemically modified nucleotide" defines a nucleotide that has a chemical modification of one or more of the heterocyclic base, sugar moiety or phosphate moiety constituents. In some embodiments, the charge of the modified nucleotide is reduced compared to DNA or RNA oligonucleotides of the same nucleobase sequence. For example, the oligonucleotide can have low negative charge, no charge, or positive charge. Modifications should not prevent, and preferably enhance, the ability of the oligonucleotides to enter a cell and carry out a function such inhibition of gene expression as discussed above.

Nucleoside analogs typically support bases capable of hydrogen bonding by Watson-Crick base-pairing to standard polynucleotide bases, where the analog backbone presents the bases in a manner to permit such hydrogen bonding in a sequence-specific fashion between the oligonucleotide analog molecule and bases in a standard polynucleotide (*e*.*g*., single-stranded RNA or single-stranded DNA). Possible analogs are those having a substantially uncharged, phosphorus-containing backbone.

Where the polynucleotide is an oligonucleotide, the oligonucleotide can be, for example, a morpholino oligonucleotide.

### X. Heterocyclic Bases

The principal naturally occurring nucleotides include uracil, thymine, cytosine, adenine and guanine as the heterocyclic bases. The oligonucleotides can include chemical modifications to their nucleobase constituents. Chemical modifications of heterocyclic bases or heterocyclic base analogs may be effective to increase the binding affinity or stability in binding a target sequence.

The modified nucleoside can be, for example, m⁵C (5-methylcytidine), m⁶A (N6-methyladenosine), s²U (2-thiouridien), ψ (pseudouridine) or Um (2-O-methyluridine). Some exemplary chemical modifications of nucleosides in the mRNA molecule further include, for example, pyridine-4-one ribonucleoside, 5-aza-uridine, 2-thio-5-aza uridine, 2-thiouridine, 4-thio pseudouridine, 2-thio pseudouridine, 5-hydroxyuridine, 3-methyluridine, 5-carboxymethyl uridine, 1-carboxymethyl pseudouridine, 5-propynyl uridine, 1-propynyl pseudouridine, 5-taurinomethyluridine, 1-taurinomethyl pseudouridine, 5-taurinomethyl-2-thio uridine, 1-taurinomethyl-4-thio uridine, 5-methyl uridine, 1-methyl pseudouridine, 4-thio-1-methyl pseudouridine, 2-thio-1-methyl pseudouridine, 1-methyl-1-deaza pseudouridine, 2-thio-1-methyl-1-deaza pseudouridine, dihydrouridine, dihydropseudouridine, 2-thio dihydrouridine, 2-thio dihydropseudouridine, 2-methoxyuridine, 2-methoxy-4-thio uridine, 4-methoxy pseudouridine, 4-methoxy-2-thio pseudouridine, 5-aza cytidine, pseudoisocytidine, 3-methyl cytidine, N4-acetylcytidine, 5-formylcytidine, N4-methylcytidine, 5-hydroxymethylcytidine, 1-methyl pseudoisocytidine, pyrrolo-cytidine, pyrrolo-pseudoisocytidine, 2-thio cytidine, 2-thio-5-methyl cytidine, 4-thio pseudoisocytidine, 4-thio-1-methyl pseudoisocytidine, 4-thio-1-methyl-1-deaza pseudoisocytidine, 1-methyl-1-deaza pseudoisocytidine, zebularine, 5-aza zebularine, 5-methyl zebularine, 5-aza-2-thio zebularine, 2-thio zebularine, 2-methoxy cytidine, 2-methoxy-5-methyl cytidine, 4-methoxy pseudoisocytidine, 4-methoxy-1-methyl pseudoisocytidine, 2-aminopurine, 2,6-diaminopurine, 7-deaza adenine, 7-deaza-8-aza adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N⁶-methyladenosine, N⁶-isopentenyladenosine, N⁶-(cis-hydroxyisopentenyl) adenosine, 2-methylthio-N⁶-(cis-hydroxyisopentenyl) adenosine, N⁶-glycinylcarbamoyladenosine, N⁶-threonylcarbamoyladenosine, 2-methylthio-N⁶-threonyl carbamoyladenosine, N⁶,N⁶-dimethyladenosine, 7-methyladenine, 2-methylthio adenine, 2-methoxy adenine, inosine, 1-methyl inosine, wyosine, wybutosine, 7-deaza guanosine, 7-deaza-8-aza guanosine, 6-thio guanosine, 6-thio-7-deaza guanosine, 6-thio-7-deaza-8-aza guanosine, 7-methyl guanosine, 6-thio-7-methyl guanosine, 7-methylinosine, 6-methoxy guanosine, 1-methylguanosine, N²-methylguanosine, N²,N²-dimethylguanosine, 8-oxo guanosine, 7-methyl-8-oxo guanosine, 1-methyl-6-thio guanosine, N²-methyl-6-thio guanosine, and N²,N²-dimethyl-6-thio guanosine. In another embodiment, the modifications are independently selected from the group consisting of 5-methylcytosine, pseudouridine and 1-methylpseudouridine.

In some embodiments, the modified nucleobase in the mRNA molecule is a modified uracil including, for example, pseudouridine (ψ), pyridine-4-one ribonucleoside, 5-aza uridine, 6-aza uridine, 2-thio-5-aza uridine, 2-thio uridine (s2U), 4-thio uridine (s4U), 4-thio pseudouridine, 2-thio pseudouridine, 5-hydroxy uridine (ho⁵U), 5-aminoallyl uridine, 5-halo uridine (e.g., 5-iodom uridine or 5-bromo uridine), 3-methyl uridine (m³U), 5-methoxy uridine (mo⁵U), uridine 5-oxyacetic acid (cmo⁵U), uridine 5-oxyacetic acid methyl ester (mcmo⁵U), 5-carboxymethyl uridine (cm⁵U), 1-carboxymethyl pseudouridine, 5-carboxyhydroxymethyl uridine (chm⁵U), 5-carboxyhydroxymethyl uridine methyl ester (mchm⁵U), 5-methoxycarbonylmethyl uridine (mcm⁵U), 5-methoxycarbonylmethyl-2-thio uridine (mcm⁵s2U), 5-aminomethyl-2-thio uridine (nm⁵s2U), 5-methylaminomethyl uridine (mnm⁵U), 5-methylaminomethyl-2-thio uridine (mnm⁵s2U), 5-methylaminomethyl-2-seleno uridine (mnm⁵se²U), 5-carbamoylmethyl uridine (ncm⁵U), 5-carboxymethylaminomethyl uridine (cmnm⁵U), 5-carboxymethylaminomethyl-2-thio uridine (cmnm⁵s2U), 5-propynyl uridine, 1-propynyl pseudouridine, 5-taurinomethyl uridine (τcm⁵U), 1-taurinomethyl pseudouridine, 5-taurinomethyl-2-thio uridine (τm⁵s2U), 1-taurinomethyl-4-thio pseudouridine, 5-methyl uridine (m⁵U, *e*.*g*., having the nucleobase deoxythymine), 1-methyl pseudouridine (m¹ψ), 5-methyl-2-thio uridine (m⁵s2U), 1-methyl-4-thio pseudouridine (m¹s⁴ψ), 4-thio-1-methyl pseudouridine, 3-methyl pseudouridine (m³ψ), 2-thio-1-methyl pseudouridine, 1-methyl-1-deaza pseudouridine, 2-thio-1-methyl-1-deaza pseudouridine, dihydrouridine (D), dihydropseudouridine, 5,6-dihydrouridine, 5-methyl dihydrouridine (m⁵D), 2-thio dihydrouridine, 2-thio dihydropseudouridine, 2-methoxy uridine, 2-methoxy-4-thio uridine, 4-methoxy pseudouridine, 4-methoxy-2-thio pseudouridine, N¹-methyl pseudouridine, 3-(3-amino-3-carboxypropyl) uridine (acp³U), 1-methyl-3-(3-amino-3-carboxypropyl) pseudouridine (acp³ψ), 5-(isopentenylaminomethyl) uridine (inm⁵U), 5-(isopentenylaminomethyl)-2-thio uridine (inm⁵s2U), .alpha-thio uridine, 2'-O-methyl uridine (Um), 5,2'-O-dimethyl uridine (m⁵Um), 2'-O-methyl pseudouridine (ψm), 2-thio-2'-O-methyl uridine (s2Um), 5-methoxycarbonylmethyl-2'-O-methyl uridine (mcm⁵Um), 5-carbamoylmethyl-2'-O-methyl uridine (ncm⁵Um), 5-carboxymethylaminomethyl-2'-O-methyl uridine (cmnm⁵Um), 3,2'-O-dimethyl uridine (m³Um), 5-(isopentenylaminomethyl)-2'-O-methyl uridine (inm⁵Um), 1-thio uridine, deoxythymidine, 2'-F-ara uridine, 2'-F uridine, 2'-OH-ara uridine, 5-(2-carbomethoxyvinyl) uridine, and 5-[3-(1-E-propenylamino) uridine.

In some embodiments, the modified nucleobase is a modified cytosine including, for example, 5-aza cytidine, 6-aza cytidine, pseudoisocytidine, 3-methyl cytidine (m³C), N⁴-acetyl cytidine (act), 5-formyl cytidine (f⁵C), N⁴-methyl cytidine (m⁴C), 5-methyl cytidine (m⁵C), 5-halo cytidine (e.g., 5-iodo cytidine), 5-hydroxymethyl cytidine (hm⁵C), 1-methyl pseudoisocytidine, pyrrolo-cytidine, pyrrolo-pseudoisocytidine, 2-thio cytidine (s2C), 2-thio-5-methyl cytidine, 4-thio pseudoisocytidine, 4-thio-1-methyl pseudoisocytidine, 4-thio-1-methyl-1-deaza pseudoisocytidine, 1-methyl-1-deaza pseudoisocytidine, zebularine, 5-aza zebularine, 5-methyl zebularine, 5-aza-2-thio zebularine, 2-thio zebularine, 2-methoxy cytidine, 2-methoxy-5-methyl cytidine, 4-methoxy pseudoisocytidine, 4-methoxy-1-methyl pseudoisocytidine, lysidine (k²C), alpha-thio cytidine, 2'-O-methyl cytidine (Cm), 5,2'-O-dimethyl cytidine (m⁵Cm), N⁴-acetyl-2'-O-methyl cytidine (ac⁴Cm), N⁴,2'-O-dimethyl cytidine (m⁴Cm), 5-formyl-2'-O-methyl cytidine (f⁵Cm), N⁴,N⁴,2'-O-trimethyl cytidine (m⁴₂Cm), 1-thio cytidine, 2'-F-ara cytidine, 2'-F cytidine, and 2'-OH-ara cytidine.

In some embodiments, the modified nucleobase is a modified adenine including, for example, 2-amino purine, 2,6-diamino purine, 2-amino-6-halo purine (*e*.*g*., 2-amino-6-chloro purine), 6-halo purine (*e*.*g*., 6-chloro purine), 2-amino-6-methyl purine, 8-azido adenosine, 7-deaza adenine, 7-deaza-8-aza adenine, 7-deaza-2-amino purine, 7-deaza-8-aza-2-amino purine, 7-deaza-2,6-diamino purine, 7-deaza-8-aza-2,6-diamino purine, 1-methyl adenosine (m¹A), 2-methyl adenine (m²A), N⁶-methyl adenosine (m⁶A), 2-methylthio-N⁶-methyl adenosine (ms²m⁶A), N⁶-isopentenyl adenosine (i⁶A), 2-methylthio-N⁶-isopentenyl adenosine (ms²i⁶A), N⁶-(cis-hydroxyisopentenyl) adenosine (io⁶A), 2-methylthio-N⁶-(cis-hydroxyisopentenyl) adenosine (ms²io⁶A), N⁶-glycinylcarbamoyl adenosine (g⁶A), N⁶-threonylcarbamoyl adenosine (t⁶A), N⁶-methyl-N⁶-threonylcarbamoyl adenosine (m⁶t⁶A), 2-methylthio-N⁶-threonylcarbamoyl adenosine (ms²g⁶A), N⁶,N⁶-dimethyl adenosine (m⁶₂A), N⁶-hydroxynorvalylcarbamoyl adenosine (hn⁶A), 2-methylthio-N⁶-hydroxynorvalylcarbamoyl adenosine (ms²hn⁶A), N⁶-acetyl adenosine (ac⁶A), 7-methyl adenine, 2-methylthio adenine, 2-methoxy adenine, alpha-thio adenosine, 2'-O-methyl adenosine (Am), N⁶,2'-O-dimethyl adenosine (m⁶Am), N⁶,N⁶,2'-O-trimethyl adenosine (m⁶₂Am), 1,2'-O-dimethyl adenosine (m¹Am), 2'-O-ribosyl adenosine (phosphate) (Ar(p)), 2-amino-N⁶-methyl purine, 1-thio adenosine, 8-azido adenosine, 2'-F-ara adenosine, 2'-F adenosine, 2'-OH-ara adenosine, and N⁶-(19-amino-pentaoxanonadecyl) adenosine.

In some embodiments, the modified nucleobase is a modified guanine including, for example, inosine (I), 1-methyl inosine (m¹I), wyosine (imG), methylwyosine (mimG), 4-demethyl wyosine (imG-14), isowyosine (imG2), wybutosine (yW), peroxywybutosine (o₂yW), hydroxywybutosine (OHyW), undermodified hydroxywybutosine (OHyWy), 7-deaza guanosine, queuosine (Q), epoxyqueuosine (oQ), galactosyl queuosine (galQ), mannosyl queuosine (manQ), 7-cyano-7-deaza guanosine (preQ₀), 7-aminomethyl-7-deaza guanosine (preQ₁), archaeosine (G⁺), 7-deaza-8-aza guanosine, 6-thio guanosine, 6-thio-7-deaza guanosine, 6-thio-7-deaza-8-aza guanosine, 7-methyl guanosine (m⁷G), 6-thio-7-methyl guanosine, 7-methyl inosine, 6-methoxy guanosine, 1-methyl guanosine (m¹G), N²-methyl-guanosine (m²G), N²,N²-dimethyl guanosine (m²₂G), N^{2,7}-dimethyl guanosine (m^{2,7}G), N², N^{2,7}-dimethyl guanosine (m^{2,2,7}G), 8-oxo guanosine, 7-methyl-8-oxo guanosine, 1-methio guanosine, N²-methyl-6-thio guanosine, N²,N²-dimethyl-6-thio guanosine, alpha-thio guanosine, 2'-O-methyl guanosine (Gm), N²-methyl-2'-O-methyl guanosine (m²Gm), N²,N²-dimethyl-2'-O-methyl guanosine (m²₂Gm), 1-methyl-2'-O-methyl guanosine (m¹Gm), N^{2,7}-dimethyl-2'-O-methyl guanosine (m^{2,7}Gm), 2'-O-methyl inosine (Im), 1,2'-O-dimethyl inosine (m¹lm), 2'-O-ribosyl guanosine (phosphate) (Gr(p)), 1-thio guanosine, O⁶-methyl guanosine, 2'-F-ara guanosine, and 2'-F guanosine.

The nucleobase of the nucleotide can be independently selected from a purine, a pyrimidine, a purine or pyrimidine analog. For example, the nucleobase can each be independently selected from adenine, cytosine, guanine, uracil or hypoxanthine. The nucleobase can also include, for example, naturally occurring and synthetic derivatives of a base, including pyrazolo[3,4-d]pyrimidines, 5-methylcytosine (5-me-C), 5-hydroxymethyl cytosine, xanthine, hypoxanthine, 2-amino adenine, 6-methyl and other alkyl derivatives of adenine and guanine, 2-propyl and other alkyl derivatives of adenine and guanine, 2-thio uracil, 2-thio thymine and 2-thio cytosine, 5-propynyl uracil and cytosine, 6-azo uracil, cytosine and thymine, pseudouracil, 4-thio uracil, 8-halo (*e*.*g*., 8-bromo), 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methyl guanine and 7-methyl adenine, 8-aza guanine and 8-aza adenine, deaza guanine, 7-deaza guanine, 3-deaza guanine, deaza adenine, 7-deaza adenine, 3-deaza adenine, pyrazolo[3,4-d]pyrimidine, imidazo[1,5-a]1,3,5 triazinones, 9-deaza purines, imidazo[4,5-d]pyrazines, thiazolo[4,5-d]pyrimidines, pyrazine-2-ones, 1,2,4-triazine, pyridazine; and 1,3,5-triazine. When the nucleotides are depicted using the shorthand A, G, C, T or U, each letter refers to the representative base and/or derivatives thereof, *e*.*g*., A includes adenine or adenine analogs, *e*.*g*., 7-deaza adenine*)*.

Other modifications include, for example, those in U.S. Patent No. 8,835,108; U.S. Patent Application Publication No. 20130156849; Tavernier, G. et al., J. Control. Release, 150:238-47, 2011; Anderson, B. et al., Nucleic Acids Res., 39:9329-38, 2011; Kormann, M. et al., Nat. Biotechnol., 29:154-7, 2011; Karikó, K. et al., Mol. Ther., 16:1833-40, 2008; Karikó, K. et al., Immunity, 23:165-75, 2005; and Warren, L. et al., Cell Stem Cell, 7:618-30, 2010; the entire contents of each of which is incorporated herein by reference.

### XI. Sugar Modifications

Polynucleotides can also contain nucleotides with modified sugar moieties or sugar moiety analogs. Sugar moiety modifications include, but are not limited to, 2'-O-aminoetoxy, 2'-O-amonioethyl (2'-OAE), 2'-β-methoxy, 2'-O-methyl, 2-guanidoethyl (2'-OGE), 2'-O,4'-C-methylene (LNA), 2'-O-(methoxyethyl) (2'-OME) and 2'-O-(N-(methyl)acetamido) (2'-OMA). 2'-O-aminoethyl sugar moiety substitutions have some advantages in certain situations as they are protonated at neutral pH and thus suppress the charge repulsion between the TFO and the target duplex. This modification stabilizes the C3'-endo conformation of the ribose or deoxyribose and also forms a bridge with the i-1 phosphate in the purine strand of the duplex.

The polynucleotide can be a morpholino oligonucleotide. Morpholino oligonucleotides are typically composed of two more morpholino monomers containing purine or pyrimidine base-pairing moieties effective to bind, by base-specific hydrogen bonding, to a base in a polynucleotide, which are linked together by phosphorus-containing linkages, one to three atoms long, joining the morpholino nitrogen of one monomer to the 5' exocyclic carbon of an adjacent monomer. The purine or pyrimidine base-pairing moiety is typically adenine, cytosine, guanine, uracil or thymine. The synthesis, structures, and binding characteristics of morpholino oligomers are detailed in U.S. Pat. Nos. 5,698,685, 5,217,866, 5,142,047, 5,034,506, 5,166,315, 5,521,063, and 5,506,337.

Important properties of the morpholino-based subunits typically include, *inter alia,* the ability to be linked in a oligomeric form by stable, uncharged backbone linkages; the ability to support a nucleotide base (*e*.*g*., adenine, cytosine, guanine, thymidine, uracil or inosine) such that the polymer formed can hybridize with a complementary base target nucleic acid, including target RNA, with high T*ₘ*, even with oligomers as short as 10-14 bases; the ability of the oligomer to be actively transported into mammalian cells; and the ability of an oligomer:RNA heteroduplex to resist RNAse degradation. In some embodiments, oligonucleotides employ morpholino-based subunits bearing base-pairing moieties, joined by uncharged linkages.

### XII. Internucleotide Linkages

Internucleotide bond refers to a chemical linkage between two nucleoside moieties. Modifications to the phosphate backbone of DNA or RNA oligonucleotides may increase the binding affinity or stability polynucleotides, or reduce the susceptibility of polynucleotides to nuclease digestion. Cationic modifications, including diethyl-ethylenediamide (DEED) or dimethyl-aminopropylamine (DMAP) may be especially useful due to decrease electrostatic repulsion between the oligonucleotide and a target. Modifications of the phosphate backbone may also include the substitution of a sulfur atom for one of the non-bridging oxygens in the phosphodiester linkage. This substitution creates a phosphorothioate internucleoside linkage in place of the phosphodiester linkage. Oligonucleotides containing phosphorothioate internucleoside linkages have been shown to be more stable *in vivo.*

Examples of modified nucleotides with reduced charge include modified internucleotide linkages such as phosphate analogs having achiral and uncharged intersubunit linkages (*e*.*g*., Stirchak, et al., J. Org. Chem., 52:4202, 1987), and uncharged morpholino-based polymers having achiral intersubunit linkages (see, *e*.*g*., U.S. Pat. No. 5,034,506). Some internucleotide linkage analogs include morpholidate, acetal, and polyamide-linked heterocycles.

In another embodiment, the oligonucleotides are composed of locked nucleic acids (LNAs). LNAs are modified RNA nucleotides (Braasch, D & Corey, D., Chem. Biol., 8:1-7, 2001). LNAs form hybrids with DNA that are more stable than DNA/DNA hybrids, a property similar to that of peptide nucleic acid (PNA)/DNA hybrids. Therefore, LNA can be used just as PNA molecules would be. LNA binding efficiency can be increased in some embodiments by adding positive charges to it. Commercial nucleic acid synthesizers and standard phosphoramidite chemistry are used to make LNAs.

In some embodiments, the oligonucleotides are composed of peptide nucleic acids. Peptide nucleic acids (PNAs) are synthetic DNA mimics in which the phosphate backbone of the oligonucleotide is replaced in its entirety by repeating N-(2-aminoethyl) glycine units and phosphodiester bonds are typically replaced by peptide bonds. The various heterocyclic bases are linked to the backbone by methylene carbonyl bonds. PNAs maintain spacing of heterocyclic bases that is similar to conventional DNA oligonucleotides, but are achiral and neutrally charged molecules. PNAs are comprised of peptide nucleic acid monomers.

Other backbone modifications include peptide and amino acid variations and modifications. Thus, the backbone constituents of oligonucleotides such as PNA may be peptide linkages, or alternatively, they may be non-peptide peptide linkages. Examples include acetyl caps, amino spacers such as 8-amino-3,6-dioxaoctanoic acid (referred to herein as O-linkers), amino acids such as lysine are particularly useful if positive charges are desired in the PNA. Methods for the chemical assembly of PNAs are well known.

Polynucleotides optionally include one or more terminal residues or modifications at either or both termini to increase stability, and/or affinity of the oligonucleotide for its target. Commonly used positively charged moieties include the amino acids lysine and arginine, although other positively charged moieties may also be useful. For example, lysine and arginine residues can be added to a bis-PNA linker or can be added to the carboxy or the N-terminus of a PNA strand. Polynucleotides may further be modified to be end-capped to prevent degradation using a 3' propylamine group. Procedures for 3' or 5' capping oligonucleotides are known in the art.

### XIII. Coating Agents for Polyplexes

Efficiency of polynucleotide delivery can be affected by the positive charges on the polyplex surface. For example, a zeta potential of the polyplex of +8.9 mV can attract and bind with negatively charged plasma proteins in the blood during circulation and lead to rapid clearance by the reticuloendothelial system (RES). Efficiency can also be affected by instability of the polyplex nanoparticles.

### a. Compositions for Altering Surface Charge

Polyplexes can be coated with an agent that is negatively charged at physiological pH. The negatively charged agent can be one, for example, that is capable of electrostatic binding to the positively charged surface of the polyplexes. The negatively charged agent can neutralize the charge of the polyplex, or reverse the charge of the polyplex. Therefore, in some embodiments, the negatively charged agent imparts a net negative charge to the polyplex.

In some embodiments, the negatively charged agent is a negatively charged polypeptide. For example, the polypeptide can include aspartic acids, glutamic acids, or a combination thereof, such that the overall charge of the polypeptide is a negative at neutral pH. In some embodiments, the polypeptide is a poly-aspartic acid polypeptide consisting of 3, 4, 5, 6, 7, 8, 9, 10, 11, 12, 13, 14, 15, 16, 17, 18, 19, 20 or more than 20 aspartic acid residues. In some embodiments, the polypeptide is a poly-glutamic acid polypeptide consisting of 3, 4, 5, 6, 7, 8, 9, 10, 11, 12, 13, 14, 15, 16, 17, 18, 19, 20 or more than 20 glutamic acid residues. Other negatively charged molecules include small molecules (e.g., MW less than 1500, 100, 750 or 500 Da) such as hyaluronic acid.

Increasing the negative charge on the surface of the particle can reduce or prevent the negative interactions described above, wherein more positively charged particles attract and bind negatively charged plasma proteins in the blood during circulation and lead to rapid clearance by the reticuloendothelial system (RES). In some embodiments, the zeta potential of the particles is from about -15 mV to about 10 mV, from about -15 mV to about 8 mV, from about -10 mV to about 8 mV or from about -8 mV to about 8 mV. The zeta potential can be more negative or more positive than the ranges above provided the particles are stable and not readily cleared from the blood stream. The zeta potential can be manipulated by coating or functionalizing the particle surface with one or more moieties that vary the surface charge. Alternatively, the monomers themselves can be functionalized and/or additional monomers can be introduced into the polymer, which vary the surface charge.

### b. Targeting Moieties

In some embodiments, the polyplexes include a cell-type or cell-state specific targeting domain or targeting signal. Examples of moieties that may be linked or unlinked to the polyplexes include, for example, targeting moieties that provide for the delivery of molecules to specific cells. The targeting signal or sequence can be specific for a host, tissue, organ, cell, organelle, non-nuclear organelle or cellular compartment. For example, the compositions disclosed herein can be modified with galactosyl-terminating macromolecules to target the compositions to the liver or to liver cells. The modified compositions selectively enter hepatocytes after interaction of the carrier galactose residues with the asialoglycoprotein receptor present in large amounts and high affinity only on these cells. Moreover, the compositions disclosed herein can be targeted to other specific intercellular regions, compartments or cell types.

In one embodiment, the targeting signal binds to its ligand or receptor, which is located on the surface of a target cell such as to bring the vector and cell membranes sufficiently close to each other to allow penetration of the vector into the cell. Additional embodiments are directed to specifically delivering polynucleotides to specific tissue or cell types, wherein the polynucleotides can encode a polypeptide or interfere with the expression of a different polynucleotide. The polynucleotides delivered to the cell can encode polypeptides that can enhance or contribute to the functioning of the cell.

The targeting moiety can be an antibody or antigen binding fragment thereof, an antibody domain, an antigen, a T-cell receptor, a cell surface receptor, a cell surface adhesion molecule, a major histocompatibility locus protein, a viral envelope protein and a peptide selected by phage display that binds specifically to a defined cell.

One skilled in the art will appreciate that the tropism of the polyplexes described can be altered by merely changing the targeting signal. It is known in the art that nearly every cell type in a tissue in a mammalian organism possesses some unique cell surface receptor or antigen. Thus, it is possible to incorporate nearly any ligand for the cell surface receptor or antigen as a targeting signal. For example, peptidyl hormones can be used as targeting moieties to target delivery to those cells that possess receptors for such hormones. Chemokines and cytokines can similarly be employed as targeting signals to target delivery of the complex to their target cells. A variety of technologies have been developed to identify genes that are preferentially expressed in certain cells or cell states and one of skill in the art can employ such technology to identify targeting signals that are preferentially or uniquely expressed on the target tissue of interest.

In one embodiment, the targeting signal is used to selectively target tumor cells. Tumor cells express cell surface markers that may only be expressed in the tumor or present in non-tumor cells but preferentially presented in tumor cells. Such markers can be targeted to increase delivery of the polyplexes to cancer cells.

In some embodiments, the targeting moiety, can be, for example, a polypeptide including an arginine-glycine-aspartic acid sequence. For example, the targeting moiety can be an arginine-glycine-aspartic acid-lysine (RGDK, mRGD) or other polypeptide that includes the RGD sequence and is capable of binding to tumor endothelium through the interaction of RGD with α*ᵥ*β₃ and α*ᵥ*β₅. In some embodiments, a targeting moiety includes the polypeptide sequence R/KxxR/K, where "x" is any amino acid that allows binding to neuropilin-1. Binding with integrins or neuropilin-1 are two approaches for improving tumor-targeted and tissue-penetrating delivery to tumors *in vivo.* Similar approaches have been reported to facilitate ligand-specific gene delivery *in vitro* and targeted gene delivery to liver, spleen, and bone marrow *in vivo.*

Other, exemplary tumor specific cell surface markers include, but are not limited to, alfa-fetoprotein (AFP), C-reactive protein (CRP), cancer antigen-50 (CA-50), cancer antigen-125 (CA-125) associated with ovarian cancer, cancer antigen 15-3 (CA15-3) associated with breast cancer, cancer antigen-19 (CA-19) and cancer antigen-242 associated with gastrointestinal cancers, carcinoembryonic antigen (CEA), carcinoma associated antigen (CAA), chromogranin A, epithelial mucin antigen (MC5), human epithelium specific antigen (HEA), Lewis(a)antigen, melanoma antigen, melanoma-associated antigens 100, 25, and 150, mucin-like carcinoma-associated antigen, multidrug resistance related protein (MRPm6), multidrug resistance related protein (MRP41), Neu oncogene protein (C-erbB-2), neuron specific enolase (NSE), P-glycoprotein (mdr1 gene product), multidrug-resistance-related antigen, p170, multidrug-resistance-related antigen, prostate specific antigen (PSA), CD56, NCAM, EGFR, CD44, and folate receptor. In one embodiment, the targeting signal consists of antibodies that are specific to the tumor cell surface markers.

Another embodiment provides an antibody or antigen binding fragment thereof bound to the disclosed polyplex acts as the targeting signal. The antibodies or antigen binding fragment thereof are useful for directing the polyplex to a cell type or cell state. In one embodiment, the polyplex is coated with a polypeptide that is an antibody binding domain, for example from a protein known to bind antibodies such as Protein A and Protein G from *Staphylococcus aureus.* Other domains known to bind antibodies are known in the art and can be substituted. The antibody binding domain links the antibody, or antigen binding fragment thereof, to the polyplex.

In certain embodiments, the antibody that serves as the targeting signal is polyclonal, monoclonal, linear, humanized, chimeric or a fragment thereof. Representative antibody fragments are those fragments that bind the antibody binding portion of the non-viral vector and include Fab, Fab', F(ab'), Fv diabodies, linear antibodies, single chain antibodies and bispecific antibodies known in the art.

In some embodiments, the targeting signal includes all or part of an antibody that directs the polyplex to the desired target cell type or cell state. Antibodies can be monoclonal or polyclonal. For human gene therapy purposes, antibodies can be derived from human genes and are specific for cell surface markers, and are produced to reduce potential immunogenicity to a human host as is known in the art. For example, transgenic mice that contain the entire human immunoglobulin gene cluster are capable of producing "human" antibodies can be utilized. In one embodiment, fragments of such human antibodies are employed as targeting signals. Single chain antibodies modeled on human antibodies can be prepared, for example, in prokaryotic culture.

In one embodiment, the targeting signal is directed to cells of the nervous system, including the brain and peripheral nervous system. Cells in the brain include several types and states and possess unique cell surface molecules specific for the type. Furthermore, cell types and states can be further characterized and grouped by the presentation of common cell surface molecules.

In one embodiment, the targeting signal is directed to specific neurotransmitter receptors expressed on the surface of cells of the nervous system. The distribution of neurotransmitter receptors is known in the art and one so skilled can direct the compositions described by using neurotransmitter receptor specific antibodies as targeting signals. Furthermore, given the tropism of neurotransmitters for their receptors, in one embodiment the targeting signal consists of a neurotransmitter or ligand capable of specifically binding to a neurotransmitter receptor.

In one embodiment, the targeting signal is specific to cells of the nervous system that may include astrocytes, microglia, neurons, oligodendrites and Schwann cells. These cells can be further divided by their function, location, shape, neurotransmitter class and pathological state. Cells of the nervous system can also be identified by their state of differentiation, for example, stem cells. Exemplary markers specific for these cell types and states are known in the art and include, but are not limited to CD133 and Neurosphere.

In one embodiment, the targeting signal is directed to cells of the musculoskeletal system. Muscle cells include several types and possess unique cell surface molecules specific for the type and state. Furthermore, cell types and states can be further characterized and grouped by the presentation of common cell surface molecules.

In one embodiment, the targeting signal is directed to specific neurotransmitter receptors expressed on the surface of muscle cells. The distribution of neurotransmitter receptors is known in the art and one so skilled can direct the compositions described by using neurotransmitter receptor specific antibodies as targeting signals. Furthermore, given the tropism of neurotransmitters for their receptors, in one embodiment the targeting signal consists of a neurotransmitter. Exemplary neurotransmitters expressed on muscle cells that can be targeted include but are not limited to acetylcholine and norepinephrine.

In one embodiment, the targeting signal is specific to muscle cells that consist of two major groupings, Type I and Type II. These cells can be further divided by their function, location, shape, myoglobin content and pathological state. Muscle cells can also be identified by their state of differentiation, for example muscle stem cells. Exemplary markers specific for these cell types and states are well known in the art include, but are not limited to, MyoD, Pax7, and MR4.

### c. Linkers

In some embodiments, the polyplex can be coated with both a negatively charged agent and a targeting moiety. In some embodiments, the negatively charged agent and the targeting moiety are linked together by a linker. The linker can be a polypeptide, or any other suitable linker that is known in the art, for example, polyethylene glycol (PEG).

In some embodiments, the linker is polypeptide that has approximately neutral charge at physiological pH. In some embodiments, the linker polypeptide is a polyglycine. For example, in some embodiments the linker consists of 3, 4, 5, 6, 7, 8, 9, 10, 11, 12, 13, 14, 15, 16, 17, 18, 19, 20, or glycine residues. In a preferred embodiment, the linker is a 6-residue polyglycine.

In some embodiments, the negatively charged agent alone, or in combination with a targeting moiety is linked to the polyplex by electrostatic interactions. In some embodiments, the negative charged agent, the targeting moiety, or a combination thereof is linked to the polyplex by covalent conjugation to the polymer backbone or to a side chain attached to the polymer backbone.

### XIV. Formulations

Formulations are prepared using a pharmaceutically acceptable "carrier" composed of materials that are considered safe and effective and may be administered to an individual without causing undesirable biological side effects or unwanted interactions. The "carrier" is all components present in the pharmaceutical formulation other than the active ingredient or ingredients. The term "carrier" includes but is not limited to diluents, binders, lubricants, disintegrators, fillers and coating compositions.

"Carrier" also includes all components of the coating composition that may include plasticizers, pigments, colorants, glidants, stabilization agents, pore formers and surfactants. Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name EUDRAGIT^{®} (Roth Pharma, Westerstadt, Germany), Zein, shellac, and polysaccharides.

Optional pharmaceutically acceptable excipients present in the drug-containing tablets, beads, granules or particles include, but are not limited to, diluents, binders, lubricants, disintegrants, colorants, stabilizers, and surfactants. Diluents, also termed "fillers," are typically necessary to increase the bulk of a solid dosage form so that a practical size is provided for compression of tablets or formation of beads and granules. Suitable diluents include, but are not limited to, dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powder sugar.

Binders are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone.

Lubricants are used to facilitate tablet manufacture. Examples of suitable lubricants include, but are not limited to, magnesium stearate, calcium stearate, stearic acid, glycerol behenate, polyethylene glycol, talc, and mineral oil.

Disintegrants are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginine, gums or cross linked polymers, such as cross-linked PVP (Polyplasdone XL from GAF Chemical Corp).

Stabilizers are used to inhibit or retard drug decomposition reactions, which include, for example, oxidative reactions.

Surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer^{®} 401, stearoyl mono isopropanolamide, and polyoxyethylene hydrogenated tallow amide. Examples of amphoteric surfactants include sodium N-dodecyl-β-alanine, sodium N-lauryl-β-iminodipropionate, myristoamphoacetate, lauryl betaine and lauryl sulfobetaine.

If desired, the tablets, beads granules or particles may also contain minor amount of nontoxic auxiliary substances such as wetting or emulsifying agents, dyes, pH buffering agents, and preservatives.

### a. Extended Release Dosage Forms

The extended release formulations are generally prepared as diffusion or osmotic systems, for example, as described in "Remington-The science and practice of pharmacy" (20th ed., Lippincott Williams & Wilkins, Baltimore, MD, 2000). A diffusion system typically consists of two types of devices, reservoir and matrix, and is known in the art. Matrix devices are generally prepared by compressing the drug with a slowly dissolving polymer carrier into a tablet form. The three major types of materials used in the preparation of matrix devices are insoluble plastics, hydrophilic polymers, and fatty compounds. Plastic matrices include, but not limited to, methyl acrylate-methyl methacrylate, polyvinyl chloride, and polyethylene. Hydrophilic polymers include, but are not limited to, methylcellulose, hydroxypropylcellulose, hydroxypropylmethylcellulose, sodium carboxymethylcellulose, and carbopol 934, polyethylene oxides. Fatty compounds include, but are not limited to, various waxes such as carnauba wax and glyceryl tristearate.

Alternatively, extended release formulations can be prepared using osmotic systems or by applying a semi-permeable coating to the dosage form. In the latter case, the desired drug release profile can be achieved by combining low permeable and high permeable coating materials in suitable proportion.

The devices with different drug release mechanisms described above could be combined in a final dosage form comprising single or multiple units. Examples of multiple units include multilayer tablets, capsules containing tablets, beads, granules, etc.

An immediate release portion can be added to the extended release system by means of either applying an immediate release layer on top of the extended release core using coating or compression process or in a multiple unit system such as a capsule containing extended and immediate release beads.

Extended release tablets containing hydrophilic polymers are prepared by techniques commonly known in the art such as direct compression, wet granulation, or dry granulation processes. Their formulations usually incorporate polymers, diluents, binders and lubricants as well as the active pharmaceutical ingredient. The usual diluents include inert powdered substances such as any of many different kinds of starch, powdered cellulose, especially crystalline and microcrystalline cellulose, sugars such as fructose, mannitol and sucrose, grain flours and similar edible powders. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders include substances such as starch, gelatin and sugars such as lactose, fructose, and glucose. Natural and synthetic gums, including acacia, alginates, methylcellulose, and polyvinylpyrrolidine can also be used. Polyethylene glycol, hydrophilic polymers, ethylcellulose and waxes can also serve as binders. A lubricant is necessary in a tablet formulation to prevent the tablet and punches from sticking in the die. The lubricant is chosen from such slippery solids as talc, magnesium and calcium stearate, stearic acid and hydrogenated vegetable oils.

Extended release tablets containing wax materials are generally prepared using methods known in the art such as a direct blend method, a congealing method, and an aqueous dispersion method. In a congealing method, the drug is mixed with a wax material and either spray-congealed or congealed and screened and processed.

### b. Delayed Release Dosage Forms

Delayed release formulations are created by coating a solid dosage form with a film of a polymer that is insoluble in the acid environment of the stomach, and soluble in the neutral environment of small intestines.

The delayed release dosage units can be prepared, for example, by coating a drug or a drug-containing composition with a selected coating material. The drug-containing composition may be, *e.g.,* a tablet for incorporation into a capsule, a tablet for use as an inner core in a "coated core" dosage form, or a plurality of drug-containing beads, particles or granules, for incorporation into either a tablet or capsule. Preferred coating materials include bioerodible, gradually hydrolyzable, gradually water-soluble, and/or enzymatically degradable polymers, and may be conventional "enteric" polymers. Enteric polymers, as will be appreciated by those skilled in the art, become soluble in the higher pH environment of the lower gastrointestinal tract or slowly erode as the dosage form passes through the gastrointestinal tract, while enzymatically degradable polymers are degraded by bacterial enzymes present in the lower gastrointestinal tract, particularly in the colon. Suitable coating materials for effecting delayed release include, but are not limited to, cellulosic polymers such as hydroxypropyl cellulose, hydroxy ethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename EUDRAGIT^{®} (including EUDRAGIT^{®} L30D-55 and L10-55 (soluble at pH 5.5 and above), EUDRAGIT^{®} L-100 (soluble at pH 6.0 and above), EUDRAGIT^{®} S (soluble at pH 7.0 and above, as a result of a higher degree of esterification), and EUDRAGIT^{®} NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylase and guar gum; zein and shellac. Combinations of different coating materials may also be used. Multi-layer coatings using different polymers may also be applied.

### c. Pulsatile Release Formulations

By "pulsatile" is meant that a plurality of drug doses are released at spaced apart intervals of time. Generally, upon ingestion of the dosage form, release of the initial dose is substantially immediate, *i.e.,* the first drug release "pulse" occurs within about one hour of ingestion. This initial pulse is followed by a first time interval (lag time) during which very little or no drug is released from the dosage form, after which a second dose is then released. Similarly, a second nearly drug release-free interval between the second and third drug release pulses may be designed. The duration of the nearly drug release-free time interval will vary depending upon the dosage form design e.g., a twice daily dosing profile, a three times daily dosing profile, etc. For dosage forms providing a twice daily dosage profile, the nearly drug release-free interval has a duration of approximately 3 hours to 14 hours between the first and second dose. For dosage forms providing a three times daily profile, the nearly drug release-free interval has a duration of approximately 2 hours to 8 hours between each of the three doses.

In one embodiment, the pulsatile release profile is achieved with dosage forms that are closed and preferably sealed capsules housing at least two drug-containing "dosage units" wherein each dosage unit within the capsule provides a different drug release profile. Control of the delayed release dosage unit(s) is accomplished by a controlled release polymer coating on the dosage unit, or by incorporation of the active agent in a controlled release polymer matrix. Each dosage unit may comprise a compressed or molded tablet, wherein each tablet within the capsule provides a different drug release profile. For dosage forms mimicking a twice a day dosing profile, a first tablet releases drug substantially immediately following ingestion of the dosage form, while a second tablet releases drug approximately 3 hours to less than 14 hours following ingestion of the dosage form. For dosage forms mimicking a three times daily dosing profile, a first tablet releases drug substantially immediately following ingestion of the dosage form, a second tablet releases drug approximately 3 hours to less than 10 hours following ingestion of the dosage form, and the third tablet releases drug at least 5 hours to approximately 18 hours following ingestion of the dosage form. It is possible that the dosage form includes more than three tablets. While the dosage form will not generally include more than a third tablet, dosage forms housing more than three tablets can be utilized.

Alternatively, each dosage unit in the capsule may comprise a plurality of drug-containing beads, granules or particles. As is known in the art, drug-containing "beads" refer to beads made with drug and one or more excipients or polymers. Drug-containing beads can be produced by applying drug to an inert support, *e*.*g*., inert sugar beads coated with drug or by creating a "core" comprising both drug and one or more excipients. Drug-containing "granules" and "particles" comprise drug particles that may or may not include one or more additional excipients or polymers. In contrast to drug-containing beads, granules and particles do not contain an inert support. Granules generally comprise drug particles and require further processing. Generally, particles are smaller than granules, and are not further processed. Although beads, granules and particles may be formulated to provide immediate release, beads and granules are generally employed to provide delayed release.

For dosage forms mimicking a twice a day dosing profile, a first group of beads, granules or particles releases drug substantially immediately following ingestion of the dosage form, while a second group of beads or granules preferably releases drug approximately 3 hours to less than 14 hours following ingestion of the dosage form. For dosage forms mimicking a three times daily dosing profile, a first group of beads, granules or particles releases drug substantially immediately following ingestion of the dosage form, a second group of beads or granules preferably releases drug approximately 3 hours to 10 hours following ingestion of the dosage form, and a third group of beads, granules or particles releases drug at least 5 hours to approximately 18 hours following ingestion of the dosage form. The above--mentioned tablets, beads, granules or particles of different drug release profiles (*e*.*g*., immediate and delayed release profiles) may be mixed and included in a capsule, tablet or matrix to provide a pulsatile dosage form having the desired release profile.

In another embodiment, the individual dosage units are compacted in a single tablet, and may represent integral but discrete segments thereof (*e*.*g*., layers), or may be present as a simple admixture. For example, drug-containing beads, granules or particles with different drug release profiles (*e*.*g*., immediate and delayed release profiles) can be compressed together into a single tablet using conventional tableting means. In a further alternative embodiment, a dosage form is provided that comprises an inner drug-containing core and at least one drug-containing layer surrounding the inner core. An outer layer of this dosage form contains an initial, immediate release dose of the drug. For dosage forms mimicking twice daily dosing, the dosage form has an outer layer that releases drug substantially immediately following oral administration and an inner core having a polymeric-coating that preferably releases the active agent approximately 3 hours to less than 14 hours following ingestion of the dosage unit. For dosage forms mimicking three times daily dosing, the dosage form has an outer layer that releases drug substantially immediately following oral administration, an inner core that preferably releases drug at least 5 hours to 18 hours following oral administration and a layer interposed between the inner core and outer layer that preferably releases drug approximately 3 hours to 10 hours following ingestion of the dosage form. The inner core of the dosage form mimicking three times daily dosing may be formulated as compressed delayed release beads or granules.

Alternatively, for dosage forms mimicking three times daily dosing, the dosage form has an outer layer and an inner layer free of drug. The outer layer releases drug substantially immediately following oral administration, and completely surrounds the inner layer. The inner layer surrounds both the second and third doses and preferably prevents release of these doses for approximately 3 hours to 10 hours following oral administration. Once released, the second dose is immediately available while the third dose is formulated as delayed release beads or granules such that release of the third dose is effected approximately 2 hours to 8 hours thereafter effectively resulting in release of the third dose at least 5 hours to approximately 18 hours following ingestion of the dosage form. The second and third doses may be formulated by admixing immediate release and delayed release beads, granules or particles and compressing the admixture to form a second and third dose-containing core followed by coating the core with a polymer coating to achieve the desired three times daily dosing profile.

In still another embodiment, a dosage form is provided that comprises a coated core-type delivery system wherein the outer layer is comprised of an immediate release dosage unit containing an active agent, such that the active agent therein is immediately released following oral administration; an intermediate layer there under which surrounds a core; and a core that is comprised of immediate release beads or granules and delayed release beads or granules, such that the second dose is provided by the immediate release beads or granules and the third dose is provided by the delayed release beads or granules.

### XV.Methods of Preparing Polyplexes

### a. Methods for Making Particles

Particles can be prepared using a variety of techniques known in the art. The technique to be used can depend on a variety of factors including the polymer used to form the nanoparticles, the desired size range of the resulting particles, and suitability for the material to be encapsulated. Suitable techniques include, but are not limited to the following:
i. Solvent Evaporation. In this method the polymer is dissolved in a volatile organic solvent. The drug (either soluble or dispersed as fine particles) is added to the solution, and the mixture is suspended in an aqueous solution that contains a surface active agent such as polyvinyl alcohol). The resulting emulsion is stirred until most of the organic solvent evaporated, leaving solid nanoparticles. The resulting nanoparticles are washed with water and dried overnight in a lyophilizer. Nanoparticles with different sizes and morphologies can be obtained by this method.
ii. Hot Melt Microencapsulation. In this method, the polymer is first melted and then mixed with the solid particles. The mixture is suspended in a non-miscible solvent (like silicon oil), and, with continuous stirring, heated to 5C. above the melting point of the polymer. Once the emulsion is stabilized, it is cooled until the polymer particles solidify. The resulting nanoparticles are washed by decantation with petroleum ether to give a free-flowing powder. The external surfaces of spheres prepared with this technique are usually smooth and dense.
iii. Solvent Removal. In this method, the drug is dispersed or dissolved in a solution of the selected polymer in a volatile organic solvent. This mixture is suspended by stirring in an organic oil (such as silicon oil) to form an emulsion. Unlike solvent evaporation, this method can be used to make nanoparticles from polymers with high melting points and different molecular weights. The external morphology of spheres produced with this technique is highly dependent on the type of polymer used.
iv. Spray-Drying. In this method, the polymer is dissolved in organic solvent. A known amount of the active drug is suspended (insoluble drugs) or co-dissolved (soluble drugs) in the polymer solution. The solution or the dispersion is then spray-dried.
v. Phase Inversion. Nanospheres can be formed from polymers using a phase inversion method wherein a polymer is dissolved in a "good" solvent, fine particles of a substance to be incorporated, such as a drug, are mixed or dissolved in the polymer solution, and the mixture is poured into a strong non-solvent for the polymer, to spontaneously produce, under favorable conditions, polymeric microspheres, wherein the polymer is either coated with the particles or the particles are dispersed in the polymer. The method can be used to produce nanoparticles in a wide range of sizes, including, for example, about 100 nanometers to about 10 microns. Substances that can be incorporated include, for example, imaging agents such as fluorescent dyes, or biologically active molecules such as proteins or nucleic acids. In the process, the polymer is dissolved in an organic solvent and then contacted with a non-solvent, which causes phase inversion of the dissolved polymer to form small spherical particles, with a narrow size distribution optionally incorporating an antigen or other substance.

Other methods known in the art that can be used to prepare nanoparticles include, but are not limited to, polyelectrolyte condensation; single and double emulsion (probe sonication); nanoparticle molding, and electrostatic self-assembly (*e*.*g*., polyethylene imine-DNA or liposomes).

In one embodiment, the loaded particles are prepared by combining a solution of the polymer, typically in an organic solvent, with the polynucleotide of interest. The polymer solution is prepared by dissolving or suspending the polymer in a solvent. The solvent should be selected so that it does not adversely affect (*e*.*g*., destabilize or degrade) the nucleic acid to be encapsulated. Suitable solvents include, but are not limited to DMSO and methylene chloride. The concentration of the polymer in the solvent can be varied as needed. In some embodiments, the concentration is for example 25 mg/mL. The polymer solution can also be diluted in a buffer, for example, sodium acetate buffer.

Next, the polymer solution is mixed with the agent to be encapsulated, such as a polynucleotide. The agent can be dissolved in a solvent to form a solution before combining it with the polymer solution. In some embodiments, the agent is dissolved in a physiological buffer before combining it with the polymer solution. The ratio of polymer solution volume to agent solution volume can be 1:1. The combination of polymer and agent are typically incubated for a few minutes to form particles before using the solution for its desired purpose, such as transfection. For example, a polymer/polynucleotide solution can be incubated for 2, 5, 10, or more than 10 minutes before using the solution for transfection. The incubation can be at room temperature. Incubation of the polymer and agent is optional, however, as polyplexes at high concentration can be used without incubation.

In some embodiments, the particles are also incubated with a solution containing a coating agent prior to use. The particle solution can be incubated with the coating agent for 2, 5, 10 or more than 10 minutes before using the polyplexes for transfection. The incubation can be at room temperature.

In some embodiments, if the agent is a polynucleotide, the polynucleotide is first complexed to a polycation before mixing with polymer. Complexation can be achieved by mixing the polynucleotides and polycations at an appropriate molar ratio. When a polyamine is used as the polycation species, it is useful to determine the molar ratio of the polyamine nitrogen to the polynucleotide phosphate (N/P ratio). Inhibitory RNAs and polyamines can be mixed together, for example, to form a complex at an N/P ratio of between approximately 1:1 to 1:25, preferably between about 8:1 to 15:1. Methods of mixing polynucleotides with polycations to condense the polynucleotide are known in the art.

The term "polycation" refers to a compound having a positive charge, preferably at least 2 positive charges, at a selected pH, preferably physiological pH. Polycationic moieties have between about 2 to about 15 positive charges, between about 2 to about 12 positive charges, or between about 2 to about 8 positive charges at selected pH values. Suitable constituents of polycations include basic amino acids and their derivatives such as arginine, asparagine, glutamine, lysine and histidine; cationic dendrimers; and amino polysaccharides. Suitable polycations can be linear, such as linear tetralysine, branched or dendrimeric in structure.

Exemplary polycations include, but are not limited to, synthetic polycations based on acrylamide and 2-acrylamido-2-methylpropanetrimethylamine, poly(N-ethyl-4-vinylpyridine) or similar quarternized polypyridine, diethylaminoethyl polymers and dextran conjugates, polymyxin B sulfate, lipopolyamines, poly(allylamines) such as the strong polycation poly(dimethyldiallylammonium chloride), polyethyleneimine, polybrene, and polypeptides such as protamine, the histone polypeptides, polylysine, polyarginine and polyornithine.

In some embodiments, the polycation is a polyamine. Polyamines are compounds having two or more primary amine groups. Suitable naturally occurring polyamines include, but are not limited to, spermine, spermidine, cadaverine and putrescine. In another embodiment, the polycation is a cyclic polyamine. Cyclic polyamines are known in the art. Exemplary cyclic polyamines include, but are not limited to, cyclen.

### b. Methods for Transfection

Transfection is carried out by contacting cells with the solution containing the polyplexes. For in vivo methods, the contacting typically occurs in vivo after the solution is administered to the subject. For in vitro methods, the solution is typically added to a culture of cells and allowed to contact the cells for minutes, hours, or days. The cells can subsequently be washed to move excess polyplexes.

### XVI. Methods of Using the Particles/Micelles

### a. Drug Delivery

The particles described herein can be used to deliver an effective amount of one or more therapeutic, diagnostic, and/or prophylactic agents to a patient in need of such treatment. The amount of agent to be administered can be readily determine by the prescribing physician and is dependent on the age and weight of the patient and the disease or disorder to be treated.

The particles are useful in drug delivery (as used herein "drug" includes therapeutic, nutritional, diagnostic and prophylactic agents), whether injected intravenously, subcutaneously, or intramuscularly, administered to the nasal or pulmonary system, injected into a tumor milieu, administered to a mucosal surface (vaginal, rectal, buccal, sublingual), or encapsulated for oral delivery. The particles may be administered as a dry powder, as an aqueous suspension (in water, saline, buffered saline, etc.), in a hydrogel, organogel, or liposome, in capsules, tablets, troches, or other standard pharmaceutical excipient.

### b. Transfection

The disclosed compositions can be for cell transfection of polynucleotides. The transfection can occur *in vitro* or *in vivo (not claimed),* and can be applied in applications including gene therapy and disease treatment. The compositions can be more efficient, less toxic, or a combination thereof when compared to a control. In some embodiments, the control is cells treated with an alternative transfection reagent such as LIPOFECTAMINE, TRANS-IT or Lipid-LNP.

The particular polynucleotide delivered by the polyplex can be selected by one of skill in the art depending on the condition or disease to be treated. The polynucleotide can be, for example, a gene or cDNA of interest, a functional nucleic acid such as an inhibitory RNA, a tRNA, an rRNA, or an mRNA, or an expression vector encoding a gene or cDNA of interest, a functional nucleic acid, a tRNA, an rRNA, or an mRNA. In some embodiments two or more polynucleotides are administered in combination.

In some embodiments, the polynucleotide is not integrated into the host cell's genome (i.e., remains extrachromosomal). Such embodiments can be useful for transient or regulated expression of the polynucleotide, and reduce the risk of insertional mutagenesis. Therefore, in some embodiments, the polyplexes are used to deliver mRNA or non-integrating expression vectors that are expressed transiently in the host cell.

In some embodiments, the polynucleotide is integrated into the host cell's genome. For example, gene therapy is a technique for correcting defective genes responsible for disease development. Researchers may use one of several approaches for correcting faulty genes: (a) a normal gene can be inserted into a nonspecific location within the genome to replace a nonfunctional gene. This approach is most common; (b) an abnormal gene can be swapped for a normal gene through homologous recombination; (c) an abnormal gene can be repaired through selective reverse mutation, which returns the gene to its normal function; (d) the regulation (the degree to which a gene is turned on or off) of a particular gene can be altered.

Gene therapy can include the use of viral vectors, for example, adenovirus, adeno-associated virus, herpes virus, vaccinia virus, polio virus, human immunodeficiency virus (HIV), neuronal trophic virus, Sindbis and other RNA viruses, including these viruses with the HIV backbone. Also useful are any viral families that share the properties of these viruses that make them suitable for use as vectors. Viral vectors typically contain nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA.

Gene targeting via target recombination, such as homologous recombination (HR), is another strategy for gene correction. Gene correction at a target locus can be mediated by donor DNA fragments homologous to the target gene. One method of targeted recombination includes the use of triplex-forming oligonucleotides (TFOs) that bind as third strands to homopurine/homopyrimidine sites in duplex DNA in a sequence-specific manner. Triplex forming oligonucleotides can interact with either double-stranded or single-stranded nucleic acids.

Non-homologous recombination, DNA repair and gene editing methods can also be used in conjunction with the aPACE terpolymers described herein. Components of non-homologous recombination, DNA repair and gene editing machinery can be provided directly or encoded by nucleic acids contained in the formulated particle comprising an aPACE terpolymer as described herein.

Methods for targeted gene therapy using triplex-forming oligonucleotides (TFO's) and peptide nucleic acids (PNAs) and their use for treating infectious diseases such as HIV have been described. The triplex-forming molecules can also be tail clamp peptide nucleic acids (tcPNAs). Highly stable PNA:DNA:PNA triplex structures can be formed from strand invasion of a duplex DNA with two PNA strands. In this complex, the PNA/DNA/PNA triple helix portion and the PNA/DNA duplex portion both produce displacement of the pyrimidine-rich triple helix, creating an altered structure that has been shown to strongly provoke the nucleotide excision repair pathway and to activate the site for recombination with the donor oligonucleotide. Two PNA strands can also be linked together to form a bis-PNA molecule.

The triplex-forming molecules are useful to induce site-specific homologous recombination in mammalian cells when used in combination with one or more donor oligonucleotides that provide the corrected sequence. Donor oligonucleotides can be tethered to triplex-forming molecules or can be separate from the triplex-forming molecules. The donor oligonucleotides can contain at least one nucleotide mutation, insertion or deletion relative to the target duplex DNA.

Double duplex-forming molecules, such as a pair of pseudocomplementary oligonucleotides, can also induce recombination with a donor oligonucleotide at a chromosomal site. Pseudocomplementary oligonucleotides are complementary oligonucleotides that contain one or more modifications such that they do not recognize or hybridize to each other, for example due to steric hindrance, but each can recognize and hybridize to complementary nucleic acid strands at the target site. In some embodiments, pseudocomplementary oligonucleotides are pseudocomplementary peptide nucleic acids (pcPNAs). Pseudocomplementary oligonucleotides can be more efficient and provide increased target site flexibility over methods of induced recombination such as triple-helix oligonucleotides and bis-peptide nucleic acids that require a polypurine sequence in the target double-stranded DNA.

### c. In Vivo Methods (not claimed)

The disclosed compositions can be used in a method of delivering polynucleotides to cells *in vivo.* It has been discovered that the disclosed aPACE polymers are more efficient and/or less toxic for systemic in vivo transfection of polynucleotides than alternative transfection reagents, including LIPOFECTAMINE, TRANS-IT, Lipid-LNP, and even other PMSCs. Accordingly, in some embodiments, the cell-specific polyplexes including a therapeutic polynucleotide are administered systemically *in vivo* to a treat a disease, for example cancer.

In some *in vivo* approaches, the compositions are administered to a subject in a therapeutically effective amount. As used herein the term "effective amount" or "therapeutically effective amount" means a dosage sufficient to treat, inhibit, or alleviate one or more symptoms of a disease or disorder being treated or to otherwise provide a desired pharmacologic and/or physiologic effect. The precise dosage will vary according to a variety of factors such as subject-dependent variables (*e*.*g*., age, immune system health, etc.), the disease, and the treatment being effected.

### d. Pharmaceutical Compositions

Pharmaceutical compositions and formulations comprising nucleic acids and, optionally, polypeptides are provided. Pharmaceutical compositions can be for administration by parenteral (intramuscular, intraperitoneal, intravenous (IV) or subcutaneous injection), transdermal (either passively or using iontophoresis or electroporation), or transmucosal (nasal, vaginal, rectal, or sublingual) routes of administration or using bioerodible inserts and can be formulated in dosage forms appropriate for each route of administration. In some embodiments, the compositions are administered systemically, for example, by intravenous or intraperitoneal administration, in an amount effective for delivery of the compositions to targeted cells. Other possible routes include trans-dermal or oral.

In certain embodiments, the compositions are administered locally, for example by injection directly into a site to be treated. In some embodiments, the compositions are injected or otherwise administered directly to one or more tumors. Typically, local injection causes an increased localized concentration of the compositions that is greater than what can be achieved by systemic administration. In some embodiments, the compositions are delivered locally to the appropriate cells by using a catheter or syringe. Other means of delivering such compositions locally to cells include using infusion pumps or incorporating the compositions into polymeric implants, which can effect a sustained release of the polyplexes to the immediate area of the implant.

The polyplexes can be provided to the cell either directly, such as by contacting it with the cell, or indirectly, such as through the action of any biological process. For example, the polyplexes can be formulated in a physiologically acceptable carrier or vehicle, and injected into a tissue or fluid surrounding the cell. The polyplexes can cross the cell membrane by simple diffusion, endocytosis, or by any active or passive transport mechanism.

The selected dosage depends upon the desired therapeutic effect, on the route of administration, and on the duration of the treatment desired. Generally, dosage levels of 0.001 to 10 mg/kg of body weight daily are administered to mammals. Generally, for intravenous injection or infusion, dosage may be lower. Generally, the total amount of the polyplex-associated nucleic acid administered to an individual will be less than the amount of the unassociated nucleic acid that must be administered for the same desired or intended effect.

### e. Formulations for Parenteral Administration

The formulation can be in the form of a suspension or emulsion. In general, pharmaceutical compositions are provided including effective amounts of nucleic acids optionally include pharmaceutically acceptable diluents, preservatives, solubilizers, emulsifiers, adjuvants and/or carriers. Such compositions include diluents sterile water, buffered saline of various buffer content (e.g., Tris-HCI, acetate, phosphate), pH and ionic strength; and optionally, additives such as detergents and solubilizing agents (*e*.*g*., TWEEN^{®} 20, TWEEN^{®} 80 also referred to as polysorbate 20 or 80), antioxidants (*e*.*g*., ascorbic acid, sodium metabisulfite), and preservatives (*e*.*g*., thimersol, benzyl alcohol) and bulking substances (*e*.*g*., lactose, mannitol).

Examples of non-aqueous solvents or vehicles are propylene glycol, polyethylene glycol, vegetable oils, such as olive oil and corn oil, gelatin, and injectable organic esters such as ethyl oleate. The formulations may be lyophilized and redissolved/resuspended immediately before use. The formulation may be sterilized by, for example, filtration through a bacteria retaining filter, by incorporating sterilizing agents into the compositions, by irradiating the compositions, or by heating the compositions.

### f. Formulations for Topical and Mucosal Administration

The polyplexes can be applied topically. Topical administration can include application to the lungs, nasal, oral (sublingual, buccal), vaginal or rectal mucosa.

Compositions can be delivered to the lungs while inhaling and traverse across the lung epithelial lining to the blood stream when delivered either as an aerosol or spray dried particles having an aerodynamic diameter of less than about 5 microns.

A wide range of mechanical devices designed for pulmonary delivery of therapeutic products can be used, including to nebulizers, metered dose inhalers, and powder inhalers, all of which are familiar to those skilled in the art. Some specific examples of commercially available devices are the Ultravent^{®} nebulizer; the Acom^{®} II nebulizer; the Ventolin^{®} metered dose inhaler; and the Spinhaler^{®} powder inhaler. Nektar, Alkermes and Mannkind all have inhalable insulin powder preparations approved or in clinical trials where the technology could be applied to the formulations described herein.

Formulations for administration to the mucosa will typically be spray dried drug particles, which may be incorporated into a tablet, gel, capsule, suspension or emulsion. Standard pharmaceutical excipients are available from any formulator. Oral formulations may be in the form of chewing gum, gel strips, tablets, capsules, or lozenges.

Transdermal formulations may also be prepared. These will typically be ointments, lotions, sprays, or patches, all of which can be prepared using standard technology. Transdermal formulations can include penetration enhancers.

### g. Co-Administration

Polyplexes disclosed herein can optionally be co--administered with one or more additional active agents. Co-administration can include the simultaneous and/or sequential administration of the one or more additional active agents and the polyplexes. The one or more additional active agents and the polyplexes can be included in the same or different pharmaceutical formulation. The one or more additional active agents and the polyplexes can achieve the same or different clinical benefit. An appropriate time course for sequential administration may be chosen by the physician, according to such factors as the nature of a patient's illness, and the patient's condition. In certain embodiments, sequential administration includes the co-administration of one or more additional active agents and the nanoparticle gene carriers within a period of one week, 72 hours, 48 hours, 24 hours or 12 hours.

The additional active agent can be chosen by the user based on the condition or disease to be treated. Examples of additional active agents include, but are not limited to, vitamin supplements, nutritional supplements, immunosuppressants, anti-viral agents, anti-bacterial agents, anti-fungal agents, anti-anxiety medication, anti-depression medication, anti-coagulants, clotting factors, anti-inflammatories, steroids such as corticosteroids, analgesic, etc.

### h. In Vitro Methods

The disclosed compositions can be used in a method of delivering polynucleotides to cells *in vitro.* For example, the polyplexes can be used for *in vitro* transfection of cells. The method typically involves contacting the cells with polyplex including a polynucleotide in an effective amount to introduce the polynucleotide into the cell's cytoplasm. In some embodiments, the polynucleotide is delivered to the cell in an effective amount to change the genotype or a phenotype of the cell. The cells can primary cells isolated from a subject, or cells of an established cell line. The cells can be of a homogenous cell type, or can be a heterogeneous mixture of different cell types. For example, the polyplexes can be introduced into the cytoplasm of cells from a heterogeneous cell line possessing cells of different types, such as in a feeder cell culture, or a mixed culture in various states of differentiation. The cells can be a transformed cell line that can be maintained indefinitely in cell culture. Exemplary cell lines are those available from American Type Culture Collection including tumor cell lines.

Any eukaryotic cell can be transfected to produce cells that express a specific nucleic acid, for example a metabolic gene, including primary cells as well as established cell lines. Suitable types of cells include but are not limited to undifferentiated or partially differentiated cells including stem cells, totipotent cells, pluripotent cells, embryonic stem cells, inner mass cells, adult stem cells, bone marrow cells, cells from umbilical cord blood, and cells derived from ectoderm, mesoderm, or endoderm. Suitable differentiated cells include somatic cells, neuronal cells, skeletal muscle, smooth muscle, pancreatic cells, liver cells, and cardiac cells. In another embodiment, siRNA, antisense polynucleotides (including siRNA or antisense polynucleotides) or inhibitory RNA can be transfected into a cell using the compositions described herein.

The methods are particularly useful in the field of personalized therapy, for example, to repair a defective gene, de-differentiate cells, or reprogram cells. For example, target cells are first isolated from a donor using methods known in the art, contacted with the polyplexes including a polynucleotide causing a change to the cell *in vitro* (*ex vivo*)*,* and administered to a patient in need thereof. Sources or cells include cells harvested directly from the patient or an allographic donor. The target cells to be administered to a subject can be, for example, autologous, *e*.*g*., derived from the subject, or syngeneic. Allogeneic cells can also be isolated from antigenically matched, genetically unrelated donors (identified through a national registry), or by using target cells obtained or derived from a genetically related sibling or parent.

Cells can be selected by positive and/or negative selection techniques. For example, antibodies binding a particular cell surface protein may be conjugated to magnetic beads and immunogenic procedures utilized to recover the desired cell type. It may be desirable to enrich the target cells prior to transient transfection. As used herein in the context of compositions enriched for a particular target cell, "enriched" indicates a proportion of a desirable element (*e*.*g*., the target cell) that is higher than that found in the natural source of the cells. A composition of cells may be enriched over a natural source of the cells by at least one order of magnitude, two or three orders of magnitude, 10, 100, 200 or 1000 orders of magnitude or more. Once target cells have been isolated, they may be propagated by growing in suitable medium according to established methods known in the art. Established cell lines may also be useful in for the methods. The cells can be stored frozen before transfection, if necessary.

Next the cells are contacted with the disclosed composition in vitro to repair, de-differentiate, re-differentiate, and/or reprogram the cell. The cells can be monitored, and the desired cell type can be selected for therapeutic administration.

Following repair, de-differentiation, and/or re-differentiation and/or reprogramming, the cells are administered to a patient in need thereof. The cells can be isolated from and administered back to the same patient, for example. In alternative embodiments, the cells are isolated from one patient and administered to a second patient. The method can also be used to produce frozen stocks of altered cells that can be stored long-term, for later use. In one embodiment, fibroblasts, keratinocytes or hematopoietic stem cells are isolated from a patient and repaired, de-differentiated, or reprogrammed *in vitro* to provide therapeutic cells for the patient.

### i. Diseases to be Treated

Embodiments of the present disclosure provide compositions and methods applicable for gene therapy protocols and the treatment of gene related diseases or disorders. It should be noted that such methods of treatment are not according to the invention as claimed.

Cell dysfunction can also be treated or reduced using the disclosed compositions and methods. In some embodiments, diseases amenable to gene therapy are specifically targeted. The disease can be in children, for example individuals less than 18 years of age, typically less than 12 years of age, or adults, for example individuals 18 years of age or more. Thus, embodiments of the present disclosure are directed to treating a host diagnosed with a disease, by transfection of the polyplex including a polynucleotide into the cell affected by the disease and wherein the polynucleotide encodes a therapeutic protein. In another embodiment, an inhibitory RNA is directed to a specific cell type or state to reduce or eliminate the expression of a protein, thereby achieving a therapeutic effect. The present disclosure encompasses manipulating, augmenting or replacing genes to treat diseases caused by genetic defects or abnormalities.

The disclosed methods and compositions can also be used to treat, manage, or reduce symptoms associated with aging, in tissue regeneration/regenerative medicine, stem cell transplantation, inducing reversible genetic modifications, expressing inhibitory RNA, cognitive enhancement, performance enhancement, and cosmetic alterations to human or non-human animals.

### j. Research Tools

In one embodiment, the present disclosure is used as a tool to investigate cellular consequences of gene expression. Mutant mice can be generated using this approach, allowing investigators to study various biological processes. More particularly, the methods and compositions disclosed herein can be used to generate cells that contain unique gene modification(s) at the discretion of one skilled in the art.

### k. Transgenic Non-Human Animals

The techniques described in the present disclosure can also be used to generate transgenic non-human animals. It should be noted that such transgenic animals are not according to the invention as claimed.

In particular, zygote microinjection, nuclear transfer, blastomere electrofusion and blastocyst injection of embryonic stem (ES) cell hybrids provide feasible strategies for creating transgenic animals. The use of cells carrying specific genes and modifications of interest allows the creation and study of the consequences of the transfected DNA. In theory, this technique offers the prospect of transferring any polynucleotide into a whole organism. For example, the disclosed methods and compositions could be used to create mice possessing the delivered polynucleotide in a specific cell type or cell state.

Another embodiment of the disclosure provides transfected non-human organisms and methods making and using them. Single or multicellular non-human organisms, *e*.*g*., mammals, *e.g.,* rodents, *e.g.,* mice, can be transfected with the compositions described herein by administering the compositions of the present disclosure to the non-human organism. In one embodiment, the polynucleotide remains episomal and does not stably integrate into the genome of the host organism. In another embodiment, the polynucleotide prevents the expression of a gene of interest. Thus, the expression of the polynucleotide in specific cells of the host can be controlled by the amount of polynucleotide administered to the host.

The disclosed transfected non-human organisms have several advantages over traditional transgenic organisms. For example, the transfected organism disclosed herein can be produced in less time that traditional transgenic organisms without sexual reproduction. Moreover, the expression of the polynucleotide of interest in the host can be directly regulated by the amount of polynucleotide of interest administered to the host. Dosage controlled expression of a polynucleotide of interest can be correlated to observed phenotypes and changes in the transfected animal. Additionally, inducible expression and/or replication control elements can be included in the polynucleotide of interest to provide inducible and dosage dependent expression and/or replication. Suitable inducible expression and/or replication control elements are known in the art. Furthermore, the effect of genes and gene modifications in specific cell types and states can be studied without affecting the entire cells of the animal.

### I. Kits

Kits or packs that supply the elements necessary to conduct transfection of eukaryotic or prokaryotic organisms, in particular the transfection of specific cell types or cell states are also disclosed. In accordance with one embodiment a kit is provided comprising the disclosed polymers, and optionally a polyplex coating, for example a target specific coating. The polymer can be combined with a polynucleotide of the user's choosing to form a complex that can be used to transfect a host or a host cell. The polyplex can be further mixed with the coating to provide cell-type or cell-state specific tropism.

The individual components of the kits can be packaged in a variety of containers, e.g., vials, tubes, microtiter well plates, bottles. Other reagents can be included in separate containers and provided with the kit; e.g., positive control samples, negative control samples, buffers, cell culture media, etc. The kits can also include instructions for use.

### EXAMPLE

Activated poly(amine-co-ester) terpolymers for EPO mRNA delivery.

A library of PACE polymers was produced to screen the different parameters that could specifically improve mRNA delivery and transfections (FIG. 1). aPACE materials were obtained by a temperature-controlled hydrolysis process. The properties of these aPACE polymers were determined (FIGS. 2A and 2B) and the aPACE terpolymers were then tested *in vitro* for Luciferase expressing mRNA transfection in HEK293 cells (FIGS. 3A and 3B) and in Daoy cells (FIG. 4). The best aPACE formulation was then tested *in vivo,* using an erythropoietin (EPO) expressing mRNA. Following intravenous administration in mice, the serum EPO levels were quantified using an ELISA assay (FIG. 5). Potential toxicity of the polyplexes was investigated after single and multiple doses by quantifying the presence of cytokines in plasma and analyzing organs structure by immunohistology. Finally, these EPO mRNA:aPACE polyplexes were administered to β-thalassemic mice, to assess the possibility to reverse the anemic status by measuring hematological parameters.

aPACE polyplexes were able to dramatically increase Luciferase expression *in vitro* compared to LIPOFECTAMINE control and non-activated PACE polymers in both HEK293 and Daoy cells (FIGS. 3A, 3B and 4). When used to deliver an mRNA coding for EPO *in vivo,* aPACE polyplexes produced sustained levels of EPO in the blood (FIG. 5), and correspondingly, the production of red blood cells and hemoglobin. This expression was not accompanied by any systemic toxicity, even after multiple injections of the mRNA:aPACE polyplexes. Thus, aPACE polymers open the way of mRNA-based treatments, allowing for reduced number of administration while maintaining a safe profile and controlling protein production.

## Claims

1. An activated polymer comprising a backbone ester prepared by a process comprising exposing the polymer to conditions such that one or more backbone esters are hydrolyzed for 1 day to 30 days or more at a temperature from 30 ± 3 degrees C to 42 ± 4 degrees C under atmospheric pressure, thereby obtaining a Mw between 5 and 10 KDa and exposing one or more activated end group(s) which are either hydroxyl or carboxylic acid end groups, and wherein the polymer is poly(amine co ester) synthesized by a method comprising combining 15 pentadecanolide (PDL), diethanolamine, and a diester/diacid selected from either diethyl sebacate (DES) or sebacic acid (SBA).

2. The activated polymer of Claim 1, wherein the synthesis is performed in the presence of a catalyst.

3. The activated polymer of Claim 1 or 2, wherein the polymer is diluted in a buffer, preferably sodium acetate buffer.

4. The activated polymer of Claim 3, wherein the synthesis is performed at about 90C for 24 ± 2 hours.

5. A microparticle, nanoparticle or combination thereof comprising the activated polymer of any of Claims 1 to 4 and one or more therapeutic, prophylactic or diagnostic agents.

6. The microparticle, nanoparticle or combination thereof of Claim 5, wherein the agent is a macromolecule or small molecule.

7. The microparticle, nanoparticle or combination thereof of Claim 6, wherein the macromolecule is a polynucleotide.

8. The microparticle, nanoparticle or combination thereof of Claim 7, wherein the polynucleotide is mRNA.

9. A method for activating a polymer comprising a backbone ester, wherein the polymer is poly(amine co ester) synthesized by a method comprising combining 15 pentadecanolide (PDL), diethanolamine, and a diester/diacid selected from either diethyl sebacate (DES) or sebacic acid (SBA), to produce a polymer suitable for delivery of an active pharmaceutical ingredient, comprising hydrolyzing one or more of the backbone esters of the polymer for 1 day to 30 days or more at a temperature from 30 ± 3 degrees C to 42 ± 4 degrees C under atmospheric pressure to obtain a Mw between 5 and 10 KDa and exposing one or more activated end group(s) which are either hydroxyl or carboxylic acid end groups.

10. A method of making an activated poly(amine co ester) polymer, comprising:
a. combining 15 pentadecanolide (PDL), diethanolamine, and a diester/diacid selected from either diethyl sebacate (DES) or sebacic acid (SBA) in the presence of a catalyst under atmospheric pressure at 90 ± 9 degrees C for 24 hours;
b. reducing the reaction pressure to 1.6 mmHg and continuing the reaction at 90 ± 9 degrees C for an additional 8 to 72 hours; and
c. hydrolyzing the terpolymers produced in b) for 1 day to 30 days or more at a temperature from 30 ± 3 degrees C to 42 ± 4 degrees C under atmospheric pressure to obtain a Mw between 5 and 10 KDa and exposing one or more activated end group(s) which are either hydroxyl or carboxylic acid end groups .

11. A macromolecule formulated in a particle comprising an activated polymer comprising one or more hydrolysed backbone esters, wherein the polymer is poly(amine co ester) synthesized by a method comprising combining 15 pentadecanolide (PDL), diethanolamine, and a diester/diacid selected from either diethyl sebacate (DES) or sebacic acid (SBA) is hydrolyzed for 1 day to 30 days or more at a temperature from 30 ± 3 degrees C to 42 ± 4 degrees C under atmospheric pressure, to obtain a Mw between 5 and 10 KDa and exposing one or more activated end group(s) which are either hydroxyl or carboxylic acid end groups, for use as a transfection agent.

12. The macromolecule of Claim 11, wherein the activated polymer is an activated poly(amine co ester).

13. The macromolecule of Claim 11, wherein the macromolecule is a polynucleotide.

14. The macromolecule of Claim 13, wherein the macromolecule is mRNA.

15. The macromolecule of Claim 11, wherein the macromolecular formulation further comprises a pharmaceutically acceptable carrier.

16. An *in-vitro* method of transfecting cells comprising contacting cells with a polynucleotide formulated with a particle comprising an activated poly(amine-co-ester) terpolymers synthesized by a method comprising combining 15 pentadecanolide (PDL), diethanolamine, and a diester/diacid selected from either diethyl sebacate (DES) or sebacic acid (SBA);and ; comprising one or more hydrolysed backbone esters that have been hydrolyzed for 1 day to 30 days or more at a temperature from 30 ± 3 degrees C to 42 ± 4 degrees C under atmospheric pressure to obtain a Mw between 5 and 10 KDa and , thereby exposing one or more activated end group(s) which are either hydroxyl or carboxylic acid end groups.

## Patentansprüche

1. Ein aktiviertes Polymer, beinhaltend einen Hauptkettenester, hergestellt durch einen Vorgang, beinhaltend das Exponieren des Polymers gegenüber derartigen Bedingungen, dass ein oder mehrere Hauptkettenester 1 Tag bis 30 Tage oder länger bei einer Temperatur von 30 ± 3 Grad C bis 42 ± 4 Grad C unter atmosphärischem Druck hydrolysiert werden, wodurch ein Mw zwischen 5 und 10 kDa erhalten wird, und Exponieren einer oder mehrerer aktivierter Endgruppen, die entweder Hydroxyl- oder Carbonsäure-Endgruppen sind, und wobei das Polymer ein durch ein Verfahren synthetisierter Poly(amincoester) ist, beinhaltend das Kombinieren von 15-Pentadecanolid (PDL), Diethanolamin und einem Diester/einer Disäure, ausgewählt aus entweder Diethylsebacat (DES) oder Sebacinsäure (SBA).

2. Aktiviertes Polymer gemäß Anspruch 1, wobei die Synthese in Gegenwart eines Katalysators durchgeführt wird.

3. Aktiviertes Polymer gemäß Anspruch 1 oder 2, wobei das Polymer in einem Puffer, vorzugsweise Natriumacetatpuffer, verdünnt ist.

4. Aktiviertes Polymer gemäß Anspruch 3, wobei die Synthese bei etwa 90 C für 24 ± 2 Stunden durchgeführt wird.

5. Ein Mikropartikel, Nanopartikel oder eine Kombination davon, beinhaltend das aktivierte Polymer gemäß einem der Ansprüche 1 bis 4 und ein oder mehrere therapeutische, prophylaktische oder diagnostische Mittel.

6. Mikropartikel, Nanopartikel oder Kombination davon gemäß Anspruch 5, wobei das Mittel ein Makromolekül oder ein kleines Molekül ist.

7. Mikropartikel, Nanopartikel oder Kombination davon gemäß Anspruch 6, wobei das Makromolekül ein Polynukleotid ist.

8. Mikropartikel, Nanopartikel oder Kombination davon gemäß Anspruch 7, wobei das Polynukleotid mRNA ist.

9. Ein Verfahren zum Aktivieren eines Polymers, beinhaltend einen Hauptkettenester, wobei das Polymer Poly(amincoester) ist, synthetisiert durch ein Verfahren, beinhaltend das Kombinieren von 15-Pentadecanolid (PDL), Diethanolamin und einem Diester/einer Disäure, ausgewählt aus entweder Diethylsebacat (DES) oder Sebacinsäure (SBA), um ein Polymer herzustellen, das zur Abgabe eines aktiven pharmazeutischen Inhaltsstoffs geeignet ist, beinhaltend das Hydrolysieren eines oder mehrerer der Hauptkettenester des Polymers für 1 Tag bis 30 Tage oder mehr bei einer Temperatur von 30 ± 3 Grad C bis 42 ± 4 Grad C unter atmosphärischem Druck, um ein Mw zwischen 5 und 10 kDa zu erhalten, und Exponieren einer oder mehrerer aktivierter Endgruppen, die entweder Hydroxyl- oder Carbonsäure-Endgruppen sind.

10. Ein Verfahren zum Anfertigen eines aktivierten Poly(amincoester)-Polymers, das Folgendes beinhaltet:
a. Kombinieren von 15-Pentadecanolid (PDL), Diethanolamin und einem Diester/einer Disäure, ausgewählt aus entweder Diethylsebacat (DES) oder Sebacinsäure (SBA), in Gegenwart eines Katalysators unter atmosphärischem Druck bei 90 ± 9 Grad C für 24 Stunden;
b. Reduzieren des Reaktionsdrucks auf 1,6 mmHg und Fortsetzen der Reaktion bei 90 ± 9 Grad C für weitere 8 bis 72 Stunden; und
c. Hydrolysieren der in b) erzeugten Terpolymere für 1 Tag bis 30 Tage oder mehr bei einer Temperatur von 30 ± 3 Grad C bis 42 ± 4 Grad C unter atmosphärischem Druck, um ein Mw zwischen 5 und 10 kDa zu erhalten, und Exponieren einer oder mehrerer aktivierter Endgruppen, die entweder Hydroxyl- oder Carbonsäure-Endgruppen sind.

11. Ein Makromolekül, das in einem Partikel formuliert ist, beinhaltend ein aktiviertes Polymer, beinhaltend einen oder mehrere hydrolysierte Hauptkettenester, wobei das Polymer Poly(amincoester) ist, synthetisiert durch ein Verfahren, beinhaltend das Kombinieren von 15-Pentadecanolid (PDL), Diethanolamin und einem Diester/einer Disäure, ausgewählt aus entweder Diethylsebacat (DES) oder Sebacinsäure (SBA), 1 Tag bis 30 Tage oder länger bei einer Temperatur von 30 ± 3 Grad C bis 42 ± 4 Grad C unter Atmosphärendruck hydrolysiert wird, um ein Mw zwischen 5 und 10 kDa zu erhalten, und Exponieren einer oder mehrerer aktivierter Endgruppen, die entweder Hydroxyl- oder Carbonsäureendgruppen sind, zur Verwendung als Transfektionsmittel.

12. Makromolekül gemäß Anspruch 11, wobei das aktivierte Polymer ein aktivierter Poly(amincoester) ist.

13. Makromolekül gemäß Anspruch 11, wobei das Makromolekül ein Polynukleotid ist.

14. Makromolekül gemäß Anspruch 13, wobei das Makromolekül mRNA ist.

15. Makromolekül gemäß Anspruch 11, wobei die makromolekulare Formulierung ferner einen pharmazeutisch akzeptablen Träger beinhaltet.

16. Ein In-vitro-Verfahren zum Transfizieren von Zellen, beinhaltend das In-Kontakt-Bringen von Zellen mit einem Polynukleotid, das mit einem Partikel formuliert ist, beinhaltend ein aktiviertes Poly(amincoester)-Terpolymer, synthetisiert durch ein Verfahren, beinhaltend das Kombinieren von 15-Pentadecanolid (PDL), Diethanolamin und einem Diester/einer Disäure, ausgewählt aus entweder Diethylsebacat (DES) oder Sebacinsäure (SBA); und beinhaltend einen oder mehrere hydrolysierte Hauptkettenester, die 1 Tag bis 30 Tage oder länger bei einer Temperatur von 30 ± 3 Grad C bis 42 ± 4 Grad C unter atmosphärischem Druck hydrolysiert wurden, um ein Mw zwischen 5 und 10 kDa zu erhalten, und dadurch Exponieren einer oder mehrerer aktivierter Endgruppen, die entweder Hydroxyl- oder Carbonsäure-Endgruppen sind.

## Revendications

1. Un polymère activé comprenant un ester de squelette préparé au moyen d'un procédé comprenant l'exposition du polymère à des conditions de telle sorte qu'un ou plusieurs esters de squelette sont hydrolysés pendant 1 jour à 30 jours ou plus à une température allant de 30 ± 3 degrés C à 42 ± 4 degrés C sous pression atmosphérique, obtenant de ce fait une Mw comprise entre 5 et 10 kDa et l'exposition d'un ou de plusieurs groupes terminaux activés qui sont des groupes terminaux soit d'hydroxyle, soit d'acide carboxylique, et le polymère étant du poly(amine-co-ester) synthétisé par une méthode comprenant la combinaison de 15-pentadécanolide (PDL), de diéthanolamine, et d'un diester/diacide sélectionné parmi soit du sébacate de diéthyle (DES), soit de l'acide sébacique (SBA).

2. Le polymère activé de la revendication 1, dans lequel la synthèse est réalisée en présence d'un catalyseur.

3. Le polymère activé de la revendication 1 ou de la revendication 2, le polymère étant dilué dans un tampon, de préférence un tampon d'acétate de sodium.

4. Le polymère activé de la revendication 3, dans lequel la synthèse est réalisée à environ 90C pendant 24 ± 2 heures.

5. Une microparticule, une nanoparticule ou une combinaison de celles-ci comprenant le polymère activé de n'importe lesquelles des revendications 1 à 4 et un ou plusieurs agents thérapeutiques, prophylactiques ou diagnostiques.

6. La microparticule, la nanoparticule ou la combinaison de celles-ci de la revendication 5, dans laquelle l'agent est une macromolécule ou une petite molécule.

7. La microparticule, la nanoparticule ou la combinaison de celles-ci de la revendication 6, dans laquelle la macromolécule est un polynucléotide.

8. La microparticule, la nanoparticule ou la combinaison de celles-ci de la revendication 7, dans laquelle le polynucléotide est de l'ARNm.

9. Une méthode visant à activer un polymère comprenant un ester de squelette, le polymère étant du poly(amine-co-ester) synthétisé au moyen d'une méthode comprenant la combinaison de 15-pentadécanolide (PDL), de diéthanolamine, et d'un diester/diacide sélectionné parmi soit du sébacate de diéthyle (DES), soit de l'acide sébacique (SBA), afin de produire un polymère convenant pour l'administration d'un ingrédient pharmaceutique actif, comprenant l'hydrolyse d'un ou de plusieurs des esters de squelette du polymère pendant 1 jour à 30 jours ou plus à une température allant de 30 ± 3 degrés C à 42 ± 4 degrés C sous pression atmosphérique afin d'obtenir une Mw comprise entre 5 et 10 kDa et l'exposition d'un ou de plusieurs groupes terminaux activés qui sont des groupes terminaux soit d'hydroxyle, soit d'acide carboxylique.

10. Une méthode pour fabriquer un polymère de poly(amine-co-ester), comprenant :
a. la combinaison de 15-pentadécanolide (PDL), de diéthanolamine, et d'un diester/diacide sélectionné parmi soit du sébacate de diéthyle (DES), soit de l'acide sébacique (SBA), en présence d'un catalyseur sous pression atmosphérique à 90 ± 9 degrés C pendant 24 heures ;
b. la réduction de la pression de réaction à 1,6 mmHg et la poursuite de la réaction à 90 ± 9 degrés C pendant 8 à 72 heures supplémentaires ; et
c. l'hydrolyse des terpolymères produits lors de b) pendant 1 jour à 30 jours ou plus à une température allant de 30 ± 3 degrés C à 42 ± 4 degrés C sous pression atmosphérique afin d'obtenir une Mw comprise entre 5 et 10 kDa et l'exposition d'un ou de plusieurs groupes terminaux activés qui sont des groupes terminaux soit d'hydroxyle, soit d'acide carboxylique.

11. Une macromolécule formulée dans une particule comprenant un polymère activé comprenant un ou plusieurs esters de squelette hydrolysés, le polymère étant du poly(amine-co-ester) synthétisé au moyen d'une méthode comprenant la combinaison de 15-pentadécanolide (PDL), de diéthanolamine, et d'un diester/diacide sélectionné parmi soit du sébacate de diéthyle (DES), soit de l'acide sébacique (SBA), est hydrolysé pendant 1 jour à 30 jours ou plus à une température allant de 30 ± 3 degrés C à 42 ± 4 degrés C sous pression atmosphérique afin d'obtenir une Mw comprise entre 5 et 10 kDa, et l'exposition d'un ou de plusieurs groupes terminaux activés qui sont des groupes terminaux soit d'hydroxyle, soit d'acide carboxylique, pour une utilisation en tant qu'agent de transfection.

12. La macromolécule de la revendication 11, dans laquelle le polymère activé est un poly(amine-co-ester) activé.

13. La macromolécule de la revendication 11, dans laquelle la macromolécule est un polynucléotide.

14. La macromolécule de la revendication 13, où la macromolécule est de l'ARNm.

15. La macromolécule de la revendication 11, dans laquelle la formulation macromoléculaire comprend en outre un support pharmaceutiquement acceptable.

16. Une méthode *in vitro* pour transfecter des cellules comprenant la mise en contact de cellules avec un polynucléotide formulé avec une particule comprenant un terpolymère de poly(amine-co-ester) activé synthétisé au moyen d'une méthode comprenant la combinaison de 15-pentadécanolide (PDL), de diéthanolamine, et d'un diester/diacide sélectionné parmi soit du sébacate de diéthyle (DES), soit de l'acide sébacique (SBA) ; et comprenant un ou plusieurs esters de squelette hydrolysés qui ont été hydrolysés pendant 1 jour à 30 jours ou plus à une température allant de 30 ± 3 degrés C à 42 ± 4 degrés C sous pression atmosphérique afin d'obtenir une Mw comprise entre 5 et 10 kDa et, de ce fait, l'exposition d'un ou de plusieurs groupes terminaux activés qui sont des groupes terminaux soit d'hydroxyle, soit d'acide carboxylique.
